# EUROPEAN PATENT SPECIFICATION

(11) **EP 1 719 466 B1**
(45) Date of publication and mention of the grant of the patent: **30.01.2019**
(21) Application number: 05710516.5
(22) Date of filing: 22.02.2005
(51) Int. Cl.: A61B 17/22

(54) **LITHOTRITE**
LITHOTRIT
LITHOTRIPTEUR

(30) Priority: 23.02.2004 JP 2004047104
(43) Date of publication of application: 08.11.2006
(73) Proprietor: Olympus Corporation, Tokyo 192-8507 (JP)
(72) Inventor: TANAKA, Kazue, Sagamihara-shi, Kanagawa 229-0038 (JP); SAKURAI, Tomohisa, Sagamihara-shi, Kanagawa 229-1124 (JP); KONISHI, Sumihito, Mitaka-shi, Tokyo 181-0011 (JP)
(74) Representative: von Hellfeld, Axel
(86) International application number: PCT/JP2005/002790
(87) International publication number: WO 2005/079677

(56) References cited:
- EP-A1- 1 380 265
- EP-A2- 1 426 014
- DE-A1- 19 609 019
- JP-A- 7 313 518
- JP-A- 7 313 518
- JP-A- 2002 095 670
- JP-A- 2002 095 670
- JP-A- 2003 245 284
- JP-A- 2003 245 284
- JP-A- 2004 041 431
- US-A- 5 391 144

## Description

### Technical Field

The present invention relates to a calculus breaking device for breaking calculi existing in the coelom of the kidney, the ureter, the bladder or the like. In the following description, the term "supersonic" should be understood as "ultrasonic".

### Background Art

Conventionally, when removing a calculus generated in the coelom of the kidney, the ureter, the bladder or the like, the calculus is broken using a supersonic calculus breaking device and an endoscope. The supersonic calculus breaking device applies supersonic wave vibration to a calculus, using a supersonic wave vibrator. Thus, the calculus is sucked while being finely broken and is discharged out of the body via a hollow probe lumen. Therefore, the supersonic wave calculus breaking device can realize an efficient operation by breaking a calculus while ensuring the safety of the living body tissue of a patient.

However, the amplitude of a supersonic wave radiated by the supersonic calculus breaking device is several tens µm and narrow. Therefore, when targeting a fairly large calculus, it takes time to break a large lump into smaller ones with still some size at first even using the supersonic wave calculus breaking device.

There is also a mechanical shock type calculus breaking device for applying a mechanical shock wave to a calculus by giving mechanical shock to the calculus . This mechanical shock type calculus breaking device can apply a large mechanical shock wave with the amplitude of approximately 1mm. Therefore, the mechanical shock type calculus breaking device is suited to break a large calculus into ones with still some size.

Therefore, as the conventional calculus breaking device, two of the mechanical shock type calculus breaking device and the supersonic wave calculus breaking device. Specifically, firstly, a calculus is broken into smaller one with still some size by the mechanical shock type calculus breaking device. Then, the smaller calculus is further finely broken by the supersonic wave calculus breaking device. Then, the finely broken calculus particles can be sucked and discharged out of the body.

However, conventionally, two of the mechanical shock type calculus breaking device and the supersonic wave calculus breaking device must be prepared and these two devices must be replaced in the midst, which is troublesome.

For the conventional calculus breaking device, a device obtained by providing one hand piece with two calculus breaking functions of a supersonic wave calculus breaking function and a mechanical shock type calculus breaking function is proposed, for example, as disclosed by Patent reference 1.

In the calculus breaking device disclosed by Patent reference 1, one hand piece is provided with both the supersonic wave calculus breaking function and the mechanical shock type calculus breaking function. Therefore, each of both the functions can be used separately without replacing one device with another. Specifically, a large calculus is broken into smaller ones with still some size by the mechanical shock type calculus breaking function. Then, this smaller broken calculus with still some size is further finely broken by the supersonic wave calculus breaking function. Then, the finely broken calculus particles can be sucked and discharged out of the body.

Patent reference 1: Publication of unexamined patent applications No. 2002-95670 Document EP 1 380 265 A1 relates to a calculus treatment apparatus for lithotripsy of a calculus. A vibration transmitting member comprises a metal hollow pipe and ultrasonic vibrations are transmitted to the vibration transmitting member. The through-hole forms a suction path for suction and evacuation of the calculus which is pulverized. A mechanical lithotripsy probe is detachably coupled and extends to and pierces through the vibration transmitting member and is projected to the outside of the distal end of the vibration transmitting member. The ultrasonic lithotripsy probe and the mechanical lithotripsy probe are arbitrarily and selectively driven by selecting and operating any of two switches in a foot switch.

Document EP 1 426 014, which is state of the art according to Article 54(3) EPC, discloses a probe which can be used as mechanical or supersonic wave probe. It also discloses electrical contacts on the handpiece, a control unit and a detection unit which detects the position of the two contacts. Dependent on their position, either the mechanical probe or the supersonic wave probe will be switched on.

### Disclosure of Invention

It is the object of the invention to provide a more efficient calculus breaking device. This object is solved by the present invention as claimed in independent claim 1. Preferred embodiments are defined by the dependent claims.

A calculus breaking device according to an aspect of the present invention comprises a supersonic wave probe for generating a supersonic wave vibration for breaking a calculus, a mechanical shock type probe for generating a mechanical shock wave, a control unit for switching between the supersonic wave probe and the mechanical shock type probe to control and drive them and a detection unit for detecting a state including hardness or a size of the calculus,wherein the control unit switches between the supersonic wave probe and the mechanical shock type probe to control and drive them, according to the detection result of the detection unit.

Another calculus breaking device according to an aspect of the present disclosure comprises a first shock type probe for generating a first shock wave for breaking a calculus, a second shock type probe for generating a second shock wave for breaking a calculus, a switching unit for switching between the first and second shock type probes, and a control unit for controlling/driving the first and second shock type probes by switching between them by the switching unit.

Another calculus breaking device according to an aspect of the present disclosure comprises a first shock type probe for generating a first shock wave for breaking a calculus, a second shock type probe for generating a second shock wave for breaking a calculus, a switching unit for switching between the first and second shock type probes, and a control unit for controlling/driving the first and second shock type probes according to the switch by the switching unit.

### Brief Description of Drawings

Fig. 1 shows the entire configuration of the calculus breaking system of the first preferred embodiment.
Fig. 2 is a perspective illustration showing the appearance of the driving device shown in Fig. 1.
Fig. 3 is a section view showing the structure of the calculus breaking probe device shown in Fig. 1.
Fig. 4 is a perspective illustration showing the appearance of the tip of the insertion unit shown in Fig. 3.
Fig. 5A is a section view showing a variation (No. 1) of the tip of the insertion unit.
Fig. 5B is a section view showing a variation (No. 2) of the tip of the insertion unit.
Fig. 6 is the internal circuit diagram of the calculus breaking device shown in Fig. 1.
Fig. 7 is the internal circuit diagram of the CPU shown in Fig. 6.
Fig. 8 is a flowchart showing the control of the CPU.
Fig. 9A is a graph showing supersonic wave output and mechanical shock output against time (No. 1).
Fig. 9B is a graph showing supersonic wave output and mechanical shock output against time (No. 2).
Fig. 9C is a graph showing supersonic wave output and mechanical shock output against time (No. 3).
Fig. 10 is a flowchart showing a variation of the control shown in Fig. 8.
Fig. 11 is the internal circuit diagram of the first variation of the calculus breaking device shown in Fig. 6.
Fig. 12 is the circuit diagram of the deflection circuit shown in Fig. 11.
Fig. 13 is the internal circuit diagram of the second variation of the driving device shown in Fig. 6.
Fig. 14 is a flowchart showing the control of the CPU shown in Fig. 13.
Fig. 15 is a graph showing supersonic wave output and mechanical shock output against time of the driving device shown in Fig. 13.
Fig. 16 is the section view of the calculus breaking probe device constituting the calculus braking system of the second preferred embodiment.
Fig. 17 is the circuit diagram of the calculus breaking device with the calculus breaking probe device shown in Fig. 16.
Fig. 18 is a flowchart showing the control of the CPU shown in Fig. 17.
Fig. 19 is a flowchart showing the control of the output availability or alarm by the CPU, according to the combined data shown in Table 1.
Fig. 20 is a flowchart showing the control of the output or alarm by the CPU, according to the combined data shown in Table 2.
Fig. 21 is the section view of the calculus breaking probe device constituting the calculus braking system of the third preferred embodiment.
Fig. 22A is the enlarged view of the switching unit in the case where the switch shown in Fig. 21 is on.
Fig. 22B is the enlarged view of the switching unit in the case where the switch shown in Fig. 21 is off.
Fig. 23 is the circuit diagram of the calculus breaking device with the calculus breaking probe device shown in Fig. 21.
Fig. 24 is a flowchart showing the control of the CPU shown in Fig. 23.
Fig. 25 is the internal circuit diagram of the variation of the calculus breaking device shown in Fig. 23.
Fig. 26 is a flowchart showing the control of the CPU shown in Fig. 25.
Fig. 27 shows the configuration of the calculus braking probe device in which a supersonic wave probe is also used as a mechanical shock type probe.
Fig. 28 shows the circuit diagram of the calculus breaking system for switching between and controlling a supersonic wave probe and a mechanical shock type probe, according to the calculus size detected from an endoscopic image.
Fig. 29 is a flowchart showing the control of the CPU shown in Fig. 28 for switching between and controlling the entry to the respective output possible states of a supersonic wave probe and a mechanical shock type probe, according to a calculus size.

### Best Mode for Carrying Out the Invention

### <The first preferred embodiment>

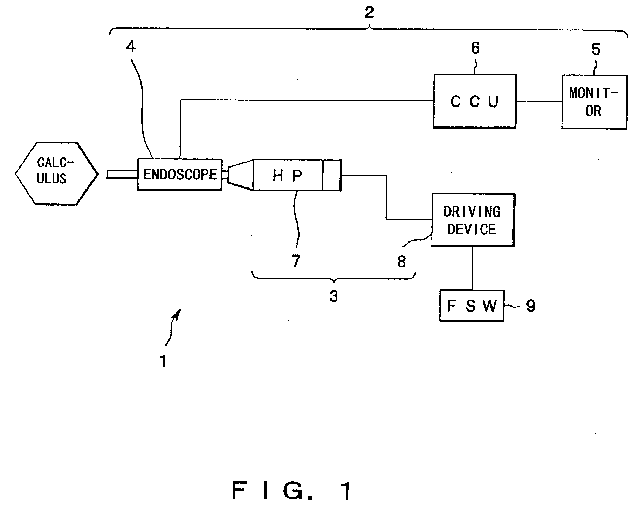

Fig. 1 shows the entire configuration of the calculus breaking system of the first preferred embodiment. As shown in Fig. 1, a calculus breaking device 1 is combined with an endoscope device 2. Furthermore, the calculus breaking device 1 comprises a calculus breaking device 3 used under the endoscope.

The endoscope device 2 comprises, for example, a hard endoscope (hereinafter simply called "endoscope") 4, a monitor 5 and a camera control unit (CCU) 6. The endoscope 4 is inserted in the coelom of a patient via a trocar, which is not shown in Fig. 1. The CCU 6 processes video signals obtained by a camera device built in this endoscope 4 and displays the Endoscopic image on the monitor 5.

The calculus breaking device 3 is led to the coelom through the treatment instrument inserting channel of the endoscope 4, which is not shown in Fig. 1. The calculus breaking device 3 comprises a calculus breaking probe device 7 and a driving device 8. The calculus breaking probe device 7 breaks calculi in the coelom. The driving device 8 controls/drives the calculus breaking probe device 7.

To the driving device 8, a foot-switch (FSW) 9 is connected. If an operator operates this foot-switch 9 by his foot (steps his foot on this foot-switch 9), a driving signal is supplied from the driving device 8 to the calculus breaking probe device 7. This driving signal drives the calculus breaking probe device 7 to break a calculus.

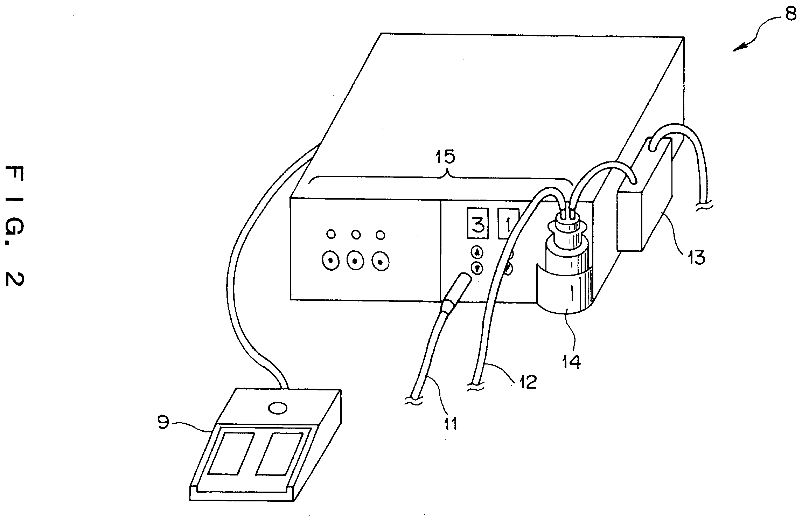

Fig. 2 is a perspective illustration showing the appearance of the driving device shown in Fig. 1. As shown in Fig. 2, a connection cable 11 for connecting the driving device 8 with the calculus breaking probe device 7 is extended from the driving device 8. A sucking tube 12 is also extended from the driving device 8. The calculus breaking probe device 7 breaks a calculus in a physiological saline solution and takes in the broken particles. The sucking tube 12 sucks the particles. A sucking pump 13 is provided on the side of the driving device 8. The sucking tube 12 sucks the broken particles from the sucking pump 13 via a sucking tank 14 and discharges the broken particles out of the body.

A setting display unit 15 is provided on the front panel of the driving device 8. The calculus breaking probe device 7 is controlled/driven by a driving mode set by the setting operation of this setting display unit 15. The detailed configuration of the driving device 8 is described later.

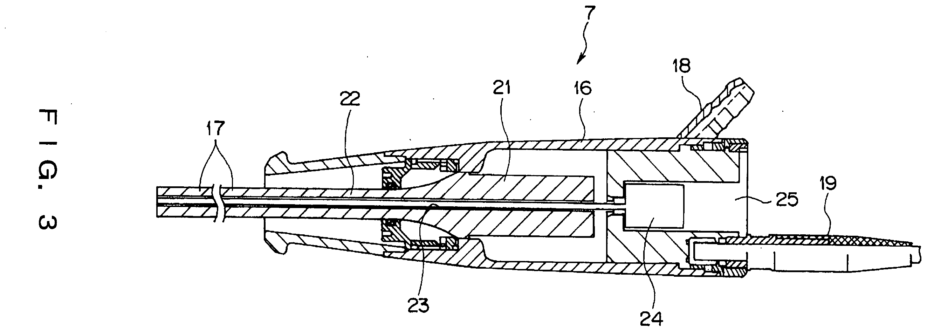

Fig. 3 is a section view showing the structure of the calculus breaking probe device shown in Fig. 1. As shown in Fig. 3, the calculus breaking probe device 7 comprises a hand piece (HP) 16 and an insertion unit 17. The hand piece (HP) 16 is a handle by firmly held by the operator. The insertion unit 17 is a slender probe to be inserted in the coelom via the treatment instrument inserting channel of the endoscope 4.

The hand piece 16 comprises a sucking mouthpiece 18 and a cable cap 19. On the sucking mouthpiece 18, the sucking tube 12 can be mounted freely attachably and detachably. On the cable mouthpiece 19, the connection cable 19 can be mounted freely attachably and detachably.

The hand piece 16 internally comprises a supersonic wave vibrator 21. In the insertion unit 17, a supersonic wave probe 22 extended from the supersonic vibrator 21 projects from the opening.

Each of the supersonic wave vibrator 21 and supersonic wave probe 22 is formed hollow and a tube path 23 is formed. At the rear of the supersonic wave vibrator 21, the rear end pf the mechanical shock type probe 24 is disposed in the accommodation unit 25. The tip of the extended probe 24 is inserted and disposed through the tube path 23 of the supersonic wave vibrator 21 and supersonic wave probe 22. Specifically, in the insertion unit 17, the mechanical shock type probe 24 is disposed in the tube path 23 of the supersonic wave probe 22.

In this case, the supersonic wave probe 22 is the first shock type probe for generating a supersonic vibration wave as the first shock wave. The mechanical shock type probe 24 is the second shock type probe for generating a mechanical shock wave as the second shock wave.

The tube path 23 of the supersonic wave vibrator 21 and supersonic wave probe 22 sucks and takes in the broken particle of the calculus from a gap between the tube path 23 and the mechanical shock type probe 24 together with a physiological saline solution by the operation of the sucking pump 13.

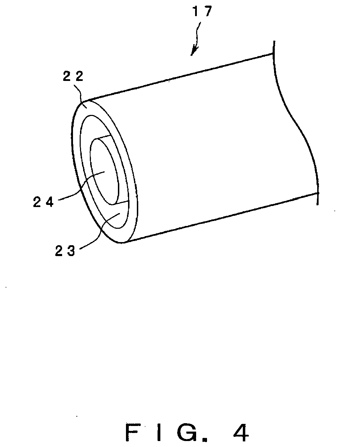

Fig. 4 is a perspective illustration showing the appearance of the tip of the insertion unit shown in Fig. 3. As shown in Fig. 4, the position of the tip of the supersonic wave probe 22 and that of the tip of the mechanical shock type probe 24 are matched. When applying the tip of the insertion unit 17 to a calculus, the respective tip of the supersonic wave probe 22 and the mechanical shock type probe 24 simultaneously touch the calculus.

The supersonic wave probe 22 and the mechanical shock type probe 24 are alternatively controlled and driven by the control/drive of the driving device 8. In this case, the mechanical shock type probe 24 advances or retreats by a stroke of approximately 1mm. The supersonic wave probe 22 vibrates at the stroke of 50∼100µm.

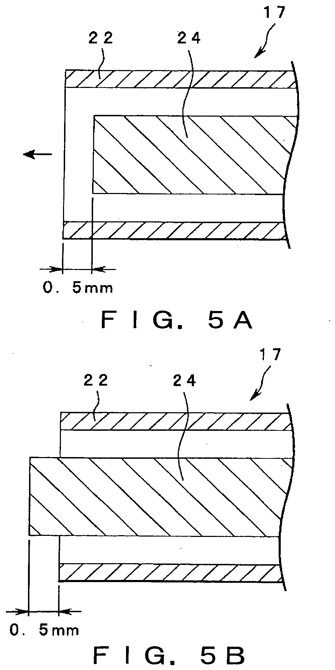

Figs. 5A and 5B are section views showing variations of the tip of the insertion unit. Fig. 5A is the section view of the tip of the insertion unit in the case where the tip of the mechanical shock type probe 24 is disposed 0.5mm, which is a half of its stroke, behind the tip of the supersonic wave probe 22. As shown in Fig. 5A, the tip of the mechanical shock type probe 24 can be disposed 0.5mm, which is a half of its stroke, behind the tip of the supersonic wave probe 22.

Fig. 5B is the section view of the tip of the insertion unit in the case where the mechanical shock type probe 24 projects 0.5mm, which is a half of its stroke, from the tip of the supersonic wave probe 22 when the mechanical shock output is turned on in the state shown in Fig. 5A. As shown in Fig. 5B, when turning the mechanical shock output on, the mechanical shock type probe 24 projects 0.5mm, which is a half of its stroke, from the tip of the supersonic wave probe 22.

As shown in Figs. 5A and 5B, when driving the supersonic wave probe 22, the mechanical shock type probe 24 does not touch a calculus and only the supersonic wave probe 22 surely touches the calculus. When driving the mechanical shock type probe 24, the supersonic wave probe 22 does not touch the calculus, and only the mechanical shock type probe 24 surely touches the calculus. Thus, the function to break a calculus by a supersonic wave and the function to break the calculus by mechanical shock can be surely used separately.

Next, the internal configuration of the driving device 8 is described.

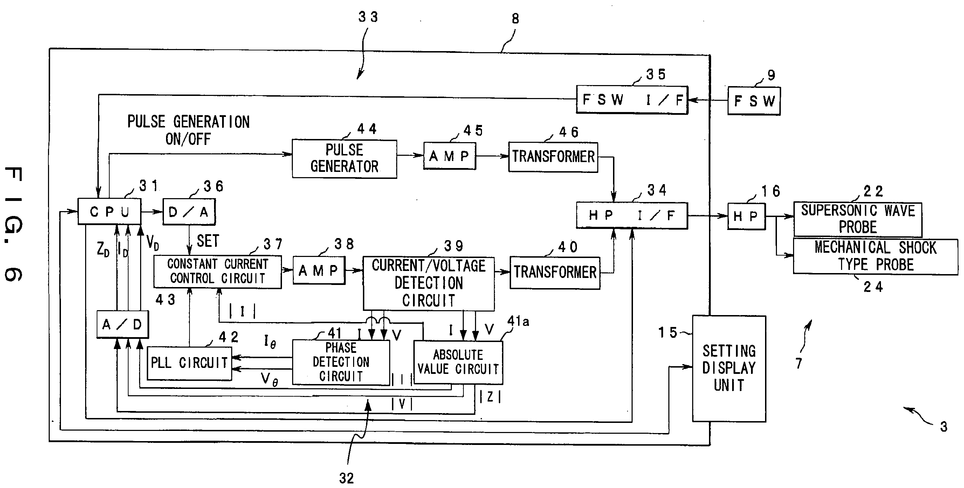

Fig. 6 is the internal circuit diagram of the calculus breaking device shown in Fig. 1. As shown in Fig. 6, the driving device 8 comprises a CPU 31, a supersonic wave output circuit 32, a mechanical shock output circuit 33 and a hand piece interface (HP I/F) 34.

The CPU 31 collectively controls each circuit, according to a driving mode selected by the operation of the setting display unit 15. The supersonic wave output circuit 32 drives the supersonic wave probe 22. The mechanical shock output circuit 33 drives the mechanical shock type probe 24. The HP I/F 34 switches between output from the supersonic wave output circuit 32 and that from the mechanical shock output circuit 33 and outputs either of them to the hand piece 16. The driving device 8 comprises a footswitch interface (FSW I/F) 35. The FSW I/F 35 takes in an on/off signal from the footswitch 9.

The supersonic wave output circuit 32 comprises a D/A converter 36, a constant current control circuit 37, an amplifier (AMP) 38, a current/voltage detection circuit 39 and a transformer 40. The constant current control circuit 37 controls voltage to perform constant current control, according to a setting signal outputted from the CPU 31 via the D/A converter 36. The AMP 38 amplifies current from the constant current control circuit 37. The current/voltage detection circuit 39 detects current (I) and voltage (V) which are amplified by the AMP 38. The transformer 40 insulates current outputted via the current/voltage detection circuit 39.

The supersonic wave output circuit 32 further comprises a phase detection circuit 41, an absolute value calculation circuit 41a, a phase locked loop (PLL) circuit 42 and an A/D converter 43. The absolute value calculation circuit 41a calculates the absolute current value (|I|), absolute voltage value (|V|) and absolute value (|Z|) of impedance Z which are detected by the current/voltage detection circuit 39. The phase detection circuit 41 detects the respective phases (I_{θ}, V_{θ}) of current and voltage which are detected by the current/voltage detection circuit 39. The PLL circuit 42 matches the respective phases of current/voltage, according to the respective phase signals of current/voltage detected by the phase detection circuit 41, in order to drive the supersonic wave vibrator 21 at a resonance point.

The absolute value calculation circuit 41a outputs the absolute current value (|I|) to the constant current control circuit 37. The absolute current value (|I|), absolute voltage value (|V|) and absolute value (|Z|) of impedance Z which are calculated by the absolute value calculation circuit 41a are digitized via the A/D converter 43. The digitized current value (I_{D}), voltage value (V_{D}) and impedance value (Z_{D}) are outputted to the CPU 31. The phase detection circuit 41 outputs the respective phase signals of the detected current/voltage to the PLL circuit 42.The constant current control circuit 37 is PLL-controlled by the PLL circuit 42.

The CPU 31 controls the on/off of the mechanical shock output circuit 33 according to the digital current/voltage values outputted from the absolute value calculation circuit 41a via the A/D converter 43.

The mechanical shock output circuit 33 comprises a pulse generation circuit 44 and an amplifier (AMP) 45 and a transformer 46. The pulse generation circuit 44 generates a pulse signal for driving the mechanical shock type probe 24, according to a pulse generation on/off signal from the CPU 31. The AMP 45 amplifies the pulse signal generated by the pulse generation circuit 44. The transformer 46 insulates the pulse signal amplified by the AMP 45.

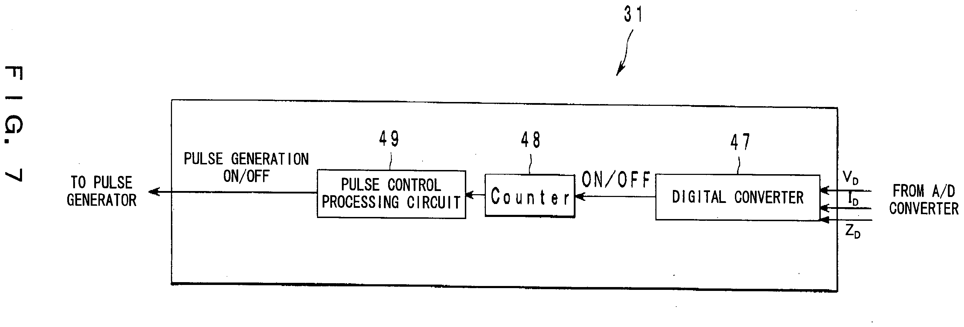

Fig. 7 is the internal circuit diagram of the CPU shown in Fig. 6. As shown in Fig. 7, the CPU 31 comprises a digital comparator 47, a counter 48 and a pulse control processing circuit 49.

The digital comparator 47 compares inputted impedance (Z_{D}) with a prescribed value T₂, which is a preset threshold value. The digital comparator 47 determines whether the calculated impedance (Z_{D}) exceeds the prescribed value T₂ and outputs an on/off signal according to the determination result. Since the impedance can also be calculated from the digital current/voltage values (I_{D}, V_{D}) inputted to the CPU 31, the impedance can also be calculated by performing the operation in the CPU 31.

The counter 48 counts the output time of an on signal from the digital comparator 47. The pulse control processing circuit 49 outputs a pulse generation on/off signal to the pulse generation circuit 44, according to the on/off signal from the digital comparator 47.

The driving device 8 switches between output from the supersonic wave output circuit 32 and that from the mechanical shock output circuit 33, based on the control of the CPU 31, according to the flowchart, which is described later, and outputs either of them to the hand piece 16. Thus, the driving device 8 switches between/controls the supersonic wave probe 22 and the mechanical shock type probe 24.

The calculus braking system so configured is used under an endoscope, as described in Fig. 1. Then, the calculus breaking probe device 7 is led to the coelom through the treatment instrument inserting channel of the endoscope 4. Then, the calculus breaking system breaks a calculus in the coelom by the control of the driving device 8.

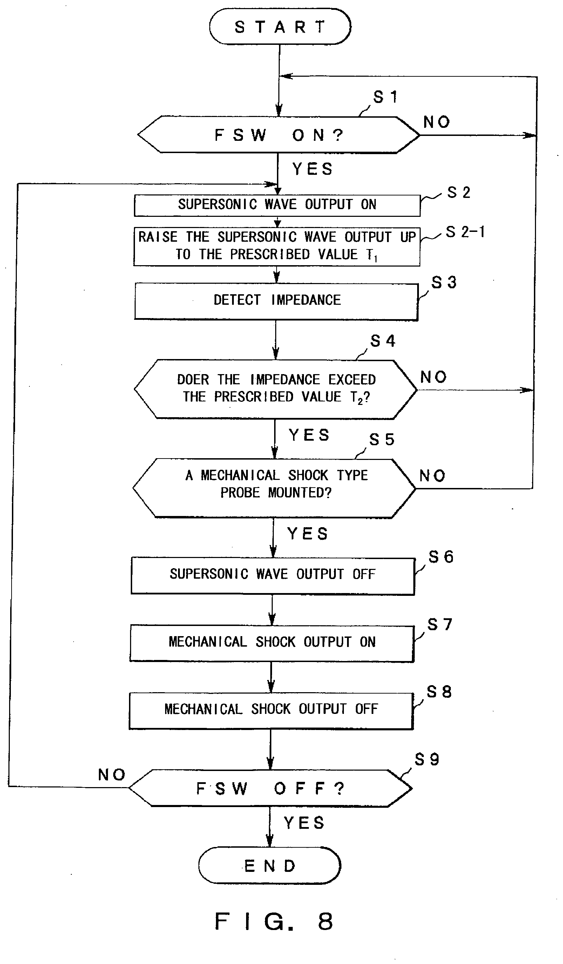

Then, the operator applies the tip of the insertion unit 17 of the calculus breaking probe device 7 to a calculus and operates the footswitch 9. Thus, the calculus is broken according to the flowchart shown in Fig. 8.

Fig. 8 is a flowchart showing the control of the CPU. As shown in Fig. 8, firstly, the CPU 31 detects whether the footswitch 9 is turned on (step S1). If the footswitch 9 is turned on, the CPU 31 controls the supersonic wave output circuit 32 to turn supersonic wave output on (step S2).

Then, in the supersonic output circuit 32, the constant current control circuit 37 performs constant current control via the D/A converter 36, according to a setting signal from the CPU 31. Then, the HP I/F 34 is switched to supply the super wave probe 22 with current. Then, in the supersonic wave output circuit 32, the AMP 38 amplifies the current outputted from the constant current control circuit 37. The transformer 40 insulates the amplified current via the current/voltage detection circuit 39 and outputs the current to the supersonic probe 22.

When current is supplied from the driving device 8, the supersonic wave vibrator 21 is driven and a supersonic wave vibration is generated. The generated supersonic wave vibration is conveyed to a probe tip to vibrate it by a supersonic wave. Then, the supersonic wave probe 22 breaks an applied calculus. At this time, in the supersonic wave output circuit 32, the current/voltage detection circuit 39 detects current/voltage. This detected current absolute value is fed back to the constant current control circuit 37. Then, the phase detection circuit 41 detects the respective phases of the current/voltage according to the current/voltage detected by the current/voltage detection circuit 39. The PLL circuit 42 PLL-controls the constant current control circuit 37, according to the respective phase signals of the detected current/voltage.

The supersonic wave output (output level) gradually rises up to a prescribed value T₁, which is a preset threshold value, under the control of the CPU 31 (step S2-1).

After the supersonic output rises up to the prescribed value T₁, the absolute value calculation circuit 41a detects a current absolute value (|I|) and a voltage absolute value (|V|), and further detects the absolute value of impedance Z (|Z|) (step S3). Furthermore, digitized current value I_{D}, voltage value V_{D} and impedance Z_{D} are outputted from the absolute value calculation circuit 41a to the CPU 31 via the A/D converter 43. In the CPU 31, the digital comparator 47 compares this inputted impedance Z_{D} with a prescribed value T₂.

Then, in the CPU 31, digital comparator 47 determines whether the impedance Z_{D} exceeds the prescribed value T₂ (step S4). If the impedance Z_{D} exceeds the prescribed value T₂, the digital comparator 47 outputs an on signal.

Simultaneously, the CPU 31 determines whether the mechanical shock type probe 24 is mounted, according to the operator's setting of the setting display unit 15 (step S5). If it is determined that the mechanical shock type probe 24 is mounted, the CPU 31 outputs a stop signal to the constant current control circuit 37. Then, the constant current control circuit 37 stops the output. Then, the supersonic wave probe 22 stops the supersonic wave vibration (turn the supersonic wave vibration off) (step S6).

Furthermore, simultaneously, in the CPU 31, the on signal is transmitted from the digital comparator 47 to the pulse control processing circuit 49 via the counter 48. This pulse control processing circuit 49 outputs a pulse generation on signal to the pulse generation circuit 44 of the mechanical shock output circuit 33. Thus, the pulse control processing circuit 49 controls the pulse generation circuit 44 to turn it on only once.

Then, the pulse generation circuit 44 generates a pulse signal. The AMP 45 amplifies this generated pulse signal. The transformer 46 insulates this amplified pulse signal. Then, the HP I/F 34 is switched and the insulated pulse signal is outputted to the mechanical shock type probe 24. Thus, the output of the mechanical shock type probe 24 is turned on (step S7). Then, the mechanical shock type probe 24 advance/retreats only once. Then, the mechanical shock type probe 24 applies a strong mechanical shock to a calculus to give a mechanical shock wave to it. Then, the output of the mechanical shock type probe 24 is turned off (step S7) .

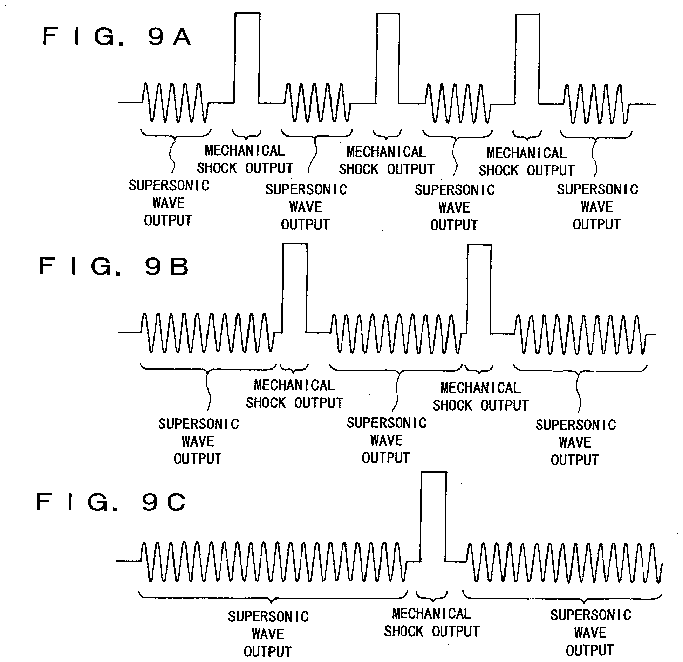

Then, the CPU 31 repeats S2 through S8 until the footswitch 9 is turned off (step S9). For example, as shown in Figs. 9A through 9C, supersonic wave output and mechanical shock output are alternatively repeated.

Figs. 9A-9C are graphs showing supersonic wave output and mechanical shock output against time. In Figs. 9A through 9C, the frequency of mechanical shock output differs due to an impedance difference depending on the state of a calculus.

Specifically, when the impedance often exceeds the prescribed value T₂ due to the hardness or size of a calculus, as shown in Fig. 9A, the frequency of mechanical shock output increases. As the frequency of a state where the impedance exceeds the prescribed value T₂ decreases, as shown in Fig. 9B, the frequency of mechanical shock output decreases. When the impedance exceeds the prescribed value T₂ only once, as shown in Fig. 9C, the mechanical shock is outputted only once.

A calculus includes a kidney calculus, a ureter calculus, a bladder calculus, urethra calculus and the like. The size of such a calculus sometimes increases up to the egg of a hen. Such a calculus cannot be sometimes broken by one mechanical shock of the mechanical shock type probe 24. Even when it is broken, sometimes its particle is still large and its impedance exceeds the prescribed value T₂. In this case, such a calculus is further finely broken by giving more mechanical shock and also giving supersonic waves until the impedance decreases below the prescribed value T₂.

As described above, in the calculus breaking system 1 of this preferred embodiment, there is no need for the operator to manually switch between the supersonic wave calculus breaking function and the mechanical shock type calculus breaking function. The calculus breaking system 1 can simultaneously control the supersonic wave calculus breaking function and the mechanical shock type calculus breaking function rapidly and safely.

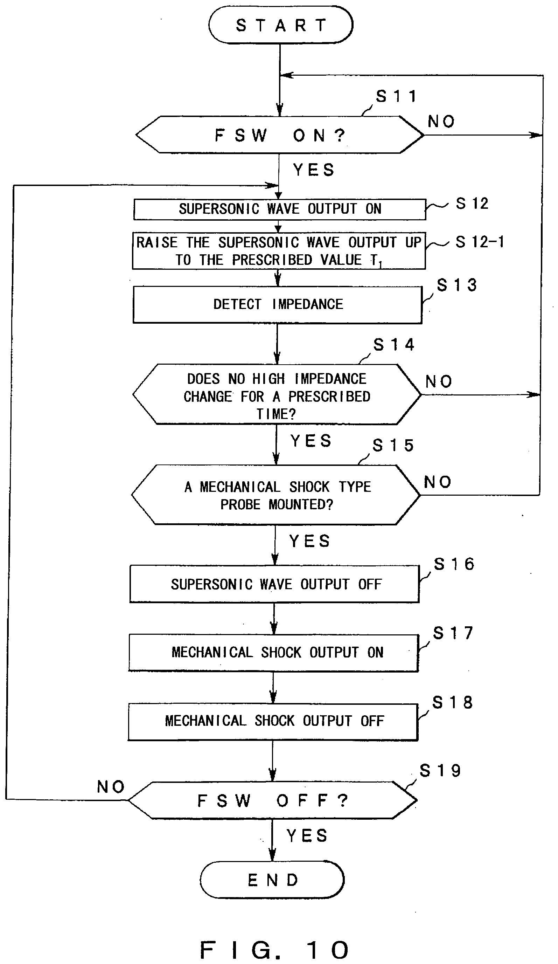

The calculus breaking system can also be configured so as to operate according to the flowchart shown in Fig. 10.

Fig. 10 is a flowchart showing a variation of the control shown in Fig. 8. The process of the flowchart shown in Fig. 10 performed until an impedance is detected (calculated) after a supersonic wave is outputted and supersonic wave vibration is applied by the supersonic wave probe 22 (S11-S13) are the same as in the flowchart shown in Fig. 8. However, Fig. 10 differs from Fig. 8 in the operation after that.

Specifically, the CPU 31 determines whether a high impedance (beyond the prescribed value T₂) changes during a prescribe preset time, according to the result of the counter 48 counting the output time of the on signal from the digital comparator 47 (step S14). If there is no change, a mechanical shock output is turned on as in the flowchart shown in Fig. 8. Then, the mechanical shock type probe 24 gives a mechanical shock to a calculus. S12 through S18 are repeated until the footswitch 9 is turned off (step S19).

Thus, the flowchart shown in Fig. 10 can obtain the same effect as in Fig. 8. In addition to this, when sometimes impedance exceeds the prescribed value T₂ even once due to accident error, the frequency in use of the mechanical shock type probe 24 is reduced and the state of a calculus can be more accurately obtained. Therefore, the supersonic wave calculus breaking function and the mechanical shock type calculus breaking function can be simultaneously controlled more efficiently.

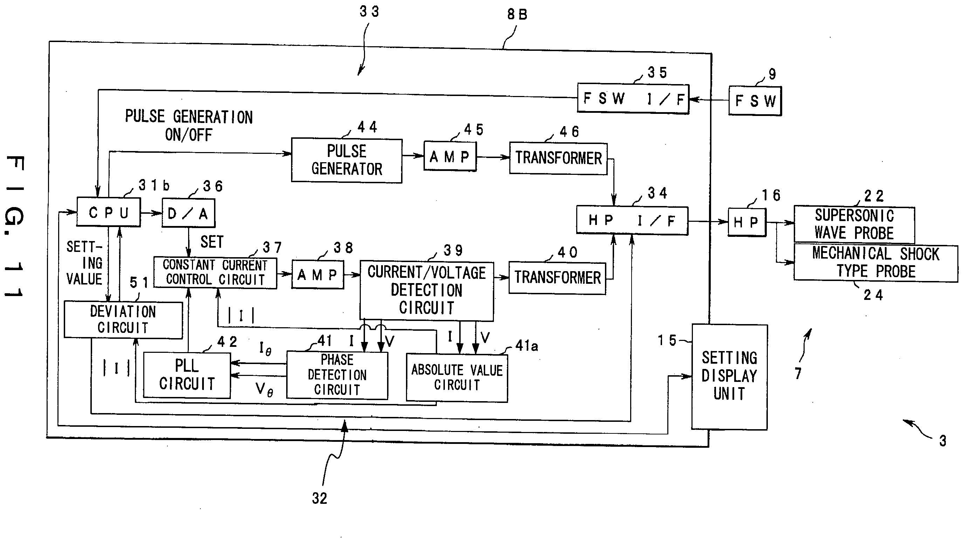

The deriving device 8 can also comprise a deflection circuit, as shown in Fig. 11.

Fig. 11 is the internal circuit diagram of the first variation of the calculus breaking device shown in Fig. 6. As shown in Fig. 11, the driving device 8B comprises a deflection circuit 51 in stead of the digital comparator 47 of the CPU 31.

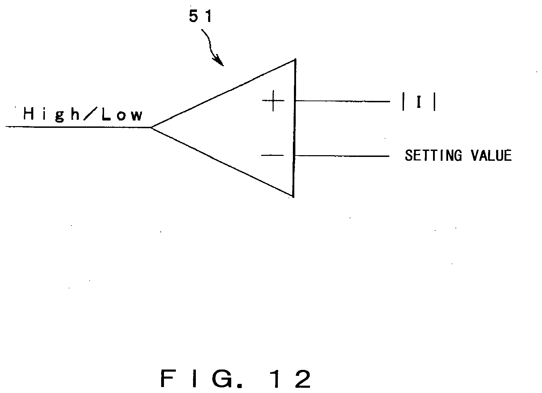

Fig. 12 is the circuit diagram of the deflection circuit shown in Fig. 11. As shown in Fig. 12, the deflection circuit receives a setting value from the CPU 31b. Then, the deflection circuit 51 compares this setting value with the absolute value of the current outputted from the absolute value calculation circuit 41a. The deflection circuit 51 outputs an on/off signal to the CPU 31b, according to the result of the comparison. The deflection circuit 51 outputs an on/off signal to the HP I/F 34 to switch it.

Thus, the driving device 8B can outputs an on/off signal by hardware without using the A/D converter 43 and without detecting impedance using the absolute value calculation circuit 41a and comparing impedance using the CPU 31b. Then, the driving device 8B can switch the HP I/F 34 by this on/off signal. Thus, the supersonic wave calculus breaking function and the mechanical shock type calculus breaking function can be simultaneously controlled more efficiently.

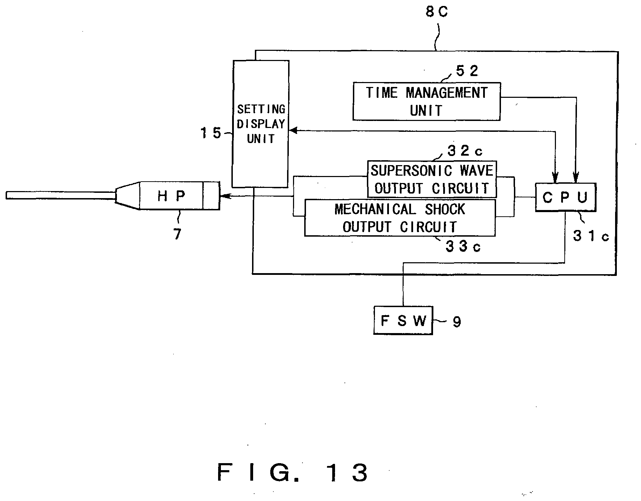

The driving device 8 can also be configured as shown in Fig. 13.

Fig. 13 is the internal circuit diagram of the second variation of the driving device shown in Fig. 6. As shown in Fig. 13, a driving device 8C comprises a CPU 31c, a supersonic wave output circuit 32c, a mechanical shock output circuit 33c and a time management unit 52. The time management unit 52, for example, manages an elapse time after the beginning of supersonic wave output.

By configuring thus, for example, when five minutes elapse after the beginning of supersonic wave output, the supersonic wave output can be stopped and switched over to mechanical shock output.

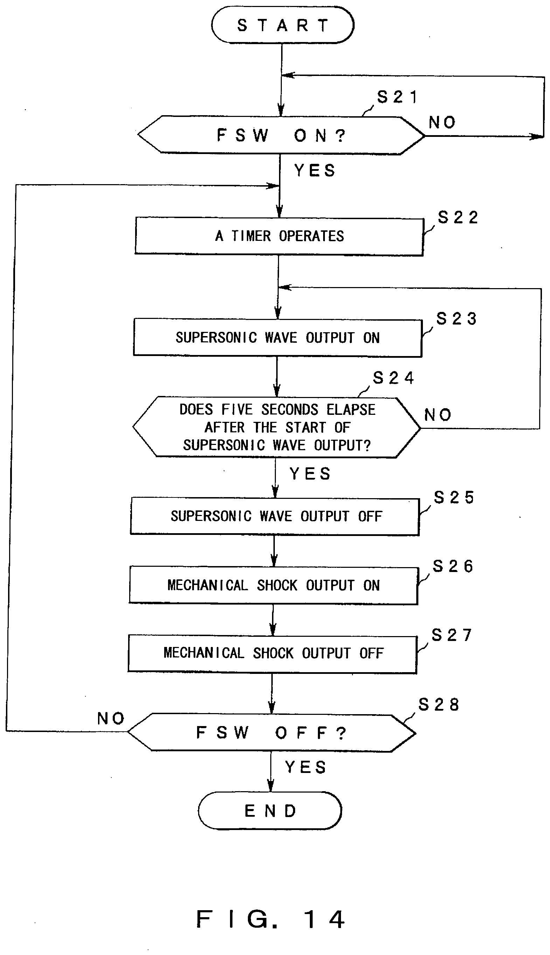

Fig. 14 is a flowchart showing the control of the CPU shown in Fig. 13. As shown in the flowchart of Fig. 14, the CPU 31c detects whether the footswitch 9 is turned on (step S21). If the footswitch 9 is turned on, the CPU 31c starts a timer, which is not shown in Fig. 13, provided for the time management unit 52 (step S22) . Simultaneously, the CPU 31c controls the supersonic wave output circuit 32c to turn supersonic wave output on (step S23). Then, the deriving device 8c supplies the supersonic wave vibrator 21 with current to drive it and to generate a supersonic wave vibration. The generated supersonic wave vibration is conveyed to a probe tip to vibrate. Thus, the supersonic probe 22 breaks an applied calculus.

The CPU 31c determines whether five seconds elapse after the beginning of supersonic wave output (step S24) . If five seconds elapse, the CPU 31c controls the supersonic wave output circuit 32c to stop supersonic wave output (turn supersonic wave output off) (step S25) . Then, the CPU 31c controls the mechanical shock output circuit 33c to turn mechanical shock output on only once (step S26). Then, the CPU 31c stops mechanical shock output (turn mechanical shock output off) (step S27) .

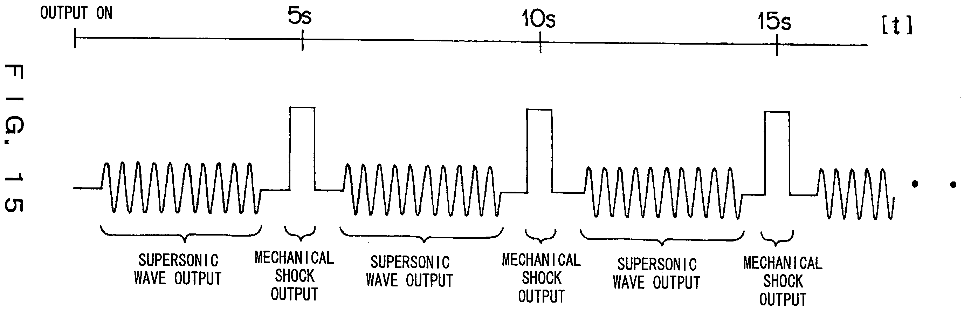

Then, the CPU 31c repeats S22 through S27 until the footswitch 9 is turned off (step S28). For example, as shown in Fig. 15, supersonic wave output and mechanical shock output are alternatively repeated every five seconds.

Fig. 15 is a graph showing supersonic wave output and mechanical shock output against time of the driving device shown in Fig. 13.

Thus, by comprising the time management unit 52, the driving device 8C can control only time lapse without detecting an impedance and current/voltage values. Thus the configuration can be simplified, and the supersonic wave calculus breaking function and the mechanical shock type calculus breaking function can be simultaneously controlled inexpensively and accurately.

### <The second preferred embodiment>

In the first preferred embodiment, the respective tips of the supersonic wave probe 22 and mechanical shock type probe 24 are disposed on the same or almost the same surface. However, in the second preferred embodiment, when mechanical shock output id turned on, the mechanical shock type probe 24 projects. Since the other components are the same as in the first preferred embodiment, their descriptions are omitted and the same reference numerals are attached to the same components.

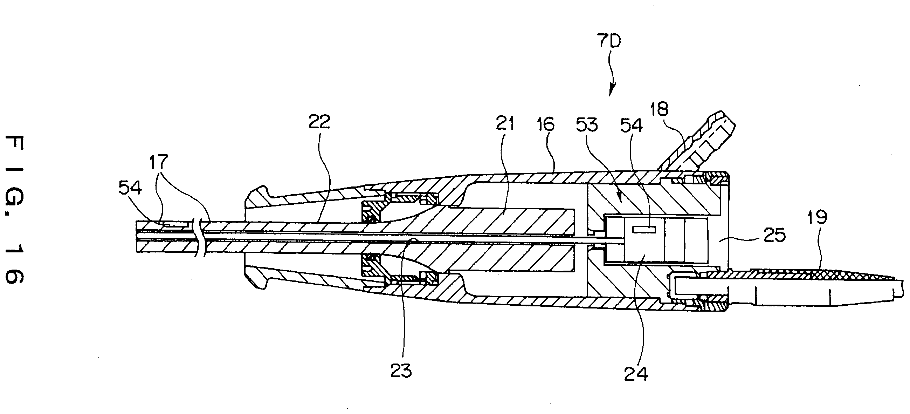

Fig. 16 is the section view of the calculus breaking probe device constituting the calculus braking system of this preferred embodiment. As shown in Fig. 16, in a calculus breaking probe device 7D, a rotary driving unit 53 is provided in the accommodation unit 25 of the hand piece 16. The rotary driving unit 53 advances/retreats the rear end of the mechanical shock type probe 24 in the longitudinal direction of a shaft.

On the rear end of the mechanical shock type probe 24, a male screw, which is not shown in Fig. 16, is formed. A female screw which is fit into this male screw and is not shown in Fig. 16 is formed on the inner circumference surface of the rotary driving unit 53. The rotary driving unit 53 is rotated by a motor or the like, which is not shown in Fig. 16. Thus, the rear end of the mechanical shock type probe 24 advance or retreats in the longitudinal direction of a shaft by the operation of the screw unit. Its probe tip freely projects against the tip of the supersonic wave probe 22.

Specifically, when the mechanical shock output is turned on, the mechanical shock type probe 24 projects against the tip of the supersonic wave probe 22. When the mechanical shock output is turned off, the mechanical shock type probe 24 retreats.

The calculus breaking probe device 7D is configured so that the supersonic wave probe 22 or mechanical shock type probe 24 can be freely attached/detached. As described later, the supersonic wave probe 22 and the mechanical shock type probe 24 can be freely combined by adjusting its probe length or probe diameter.

Therefore, in this preferred embodiment, it must be determined whether the supersonic wave probe 22 and the mechanical shock type probe 24 are properly combined. A radio frequency identification (RFID) tag 54 is attached as a storage medium for storing information about the respective probe lengths and probe diameters of the supersonic wave probe 22 and mechanical shock type probe 24.

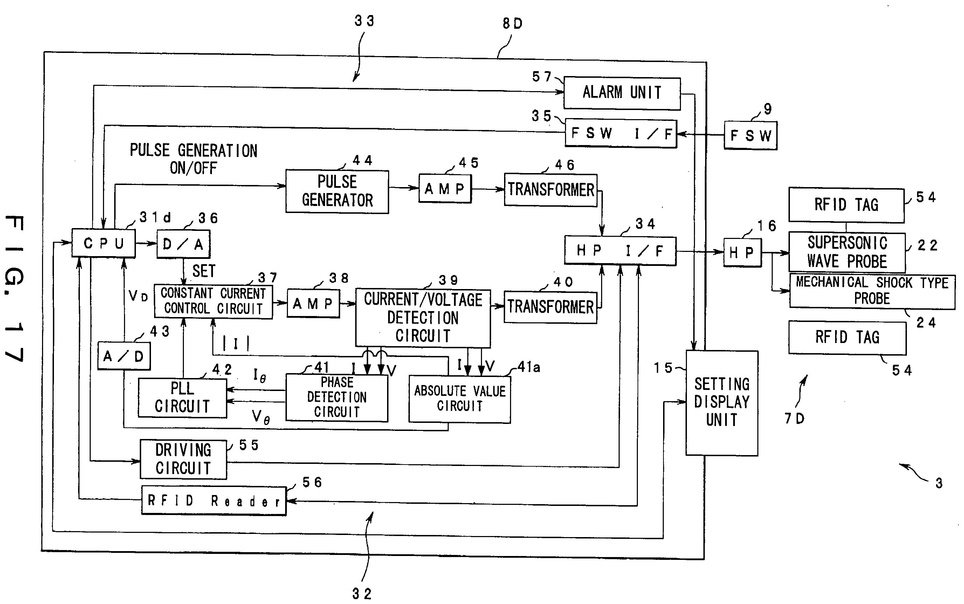

The driving device 8D for controlling/driving the calculus breaking probe device 7D is configured as shown in Fig. 17.

Fig. 17 is the circuit diagram of the calculus breaking device with the calculus breaking probe device shown in Fig. 16. As shown in Fig. 17, the driving device 8D further comprises a driving circuit 55, an RFID reader 56 and an alarm unit 57.

The driving circuit 55 drives the rotary driving unit 53 of the calculus breaking probe device 7D. The RFID reader 56 reads the information of the respective RFID tags 54 attached to the supersonic wave probe 22 and the mechanical shock type probe 24. The CPU 31d performs a prescribed determination, according to information read by the RFID reader 56. The alarm unit 57 issues an alarm, according to the determination result of the CPU 31d. Since the other components are the same as in the first preferred embodiment, their descriptions are omitted here.

The calculus breaking system configured thus is used under an endoscope as in the first preferred embodiment. In this calculus breaking system, the calculus breaking probe device 7D is led into the coelom through the treatment instrument inserting channel of the endoscope 4. Thus, the calculus breaking probe device 7D breaks a calculus in the coelom under the control of the driving device 8D.

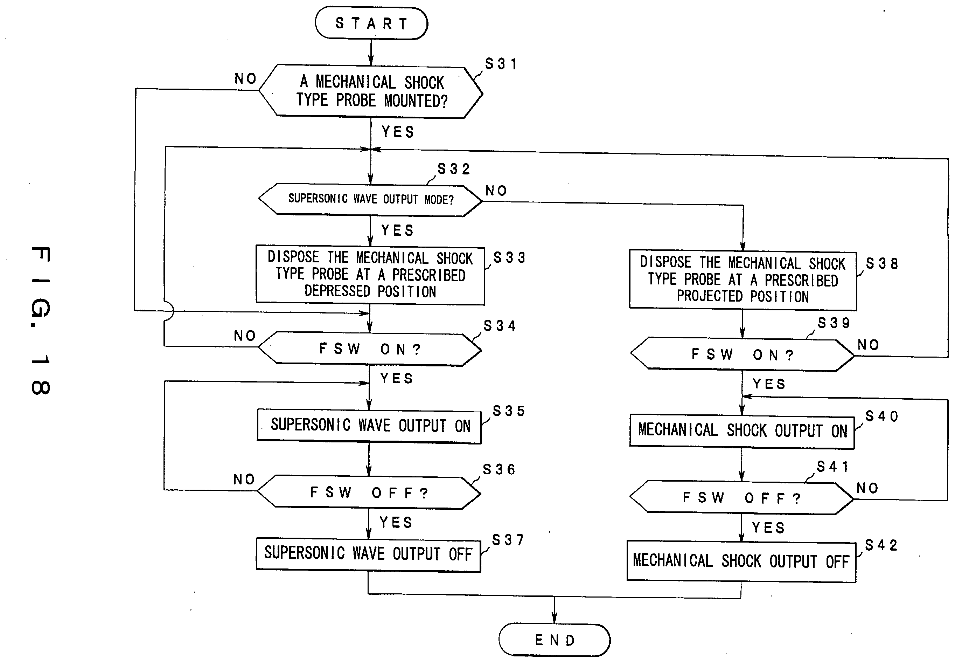

Fig. 18 is a flowchart showing the control of the CPU shown in Fig. 17. As shown in Fig. 18, firstly, the CPU 31d determines whether the mechanical shock type probe 24 is mounted, according to information from the RFID tag 54 (step S31). If the mechanical shock type probe 24 is mounted, the process proceeds to a subsequent step.

Then, the CPU 31d determines whether a supersonic wave output mode is selected, according to the operator's setting of the setting display unit 15 (step S32) . If a supersonic wave output mode is selected, the CPU 31d controls the driving circuit55 to drive the rotary driving unit 53 and to dispose the mechanical shock type probe 24 at a depressed (retreated) position (step S33).

Then, the operator applies the tip of the insertion unit 17 of the calculus breaking probe device 7D and operates the footswitch 9. Thus, a calculus is broken by supersonic wave output.

The CPU 31d detects whether the footswitch 9 is turned on (step S34). If the footswitch 9 is turned on, the CPU 31d controls the supersonic wave output circuit 32 to turn supersonic wave output on (step S35).

Then, the driving device 8D supplies the supersonic wave vibrator 21 with current. Then, the supersonic vibrator 21 is driven and supersonic wave vibration is generated. The generated supersonic wave vibration is conveyed to a probe tip to vibrate it. Thus, the supersonic wave probe 22 breaks an applied calculus . If the footswitch 9 is not turned on, the CPU 31d returns to step S32.

Then, the CPU 31d continues the supersonic wave output until the footswitch 9 is turned off (step S36) and the supersonic wave output is turned off (step S37) . When the footswitch 9 is turned off, the CPU 31d turns the supersonic wave output off to terminate.

If a supersonic wave output mode is not selected, the CPU 31d controls the driving circuit 55 to drive the rotary driving unit 53 and to dispose the mechanical shock type probe 24 in a projected position (step S38) .

Then, the operator applies the tip of the insertion unit 17 of the calculus breaking probe device 7D to calculus and operates the footswitch 9. Thus, the calculus is broken by mechanical shock output.

The CPU 31d detects whether the footswitch 9 is turned on (step S39). If the footswitch 9 is turned on, the CPU 31d controls the mechanical shock output circuit 33 to turn mechanical shock output on (step S40).

Then, the driving device 8D supplies the mechanical shock type probe 24 with a pulse signal, and the mechanical shock type probe 24 advances/retreats. Then, the mechanical shock probe 24 gives a strong mechanical shock to a calculus to give a mechanical shock wave to it and to break the calculus. If the footswitch 9 is not turned on, the CPU 31d returns to step S32.

Then, the CPU 31d continues the mechanical shock output until the footswitch 9 is turned off (step S41) and the mechanical shock output is turned off (step S42) . When the footswitch 9 is turned off, the CPU 31d turns the mechanical shock output off to terminate.

Thus, the calculus breaking system of the second preferred embodiment can obtain the same effect as in the first preferred embodiment. In addition to this, the supersonic wave calculus breaking function and the mechanical shock type calculus breaking function can be switched between by the selection of a supersonic wave output mode.

The supersonic wave probe 22 and the mechanical shock type probe 24 can be, for example, combined as shown in Table 1.

**[Table 1]**

| Inner diameter of supersonic wave probe | Outer diameter of mechanical shock type probe | | |
|---|---|---|---|
| | *φ* 0.8 | *φ* 1.2 | *φ* 2.0 |
| *φ* 1.4 | ○ | × | × |
| *φ* 2.2 | ○ | ○ | × |
| *φ* 3.2 | ○ | ○ | ○ |

| | | | |
|---|---|---|---|
| ○ : Output available × : Output unavailable | | | |

Table 1 particularly shows the outer diameters of the mechanical shock type probe 24 which can or cannot output against the inner diameters of the supersonic wave probe 22. This table shows a case that three outer diameters of the mechanical shock type probe 24, φ0.8mm, 1.2mm and 2.0mm are used against the inner diameters of the supersonic wave probe 22, φ1.4mm, 2.2mm and 3.0mm.

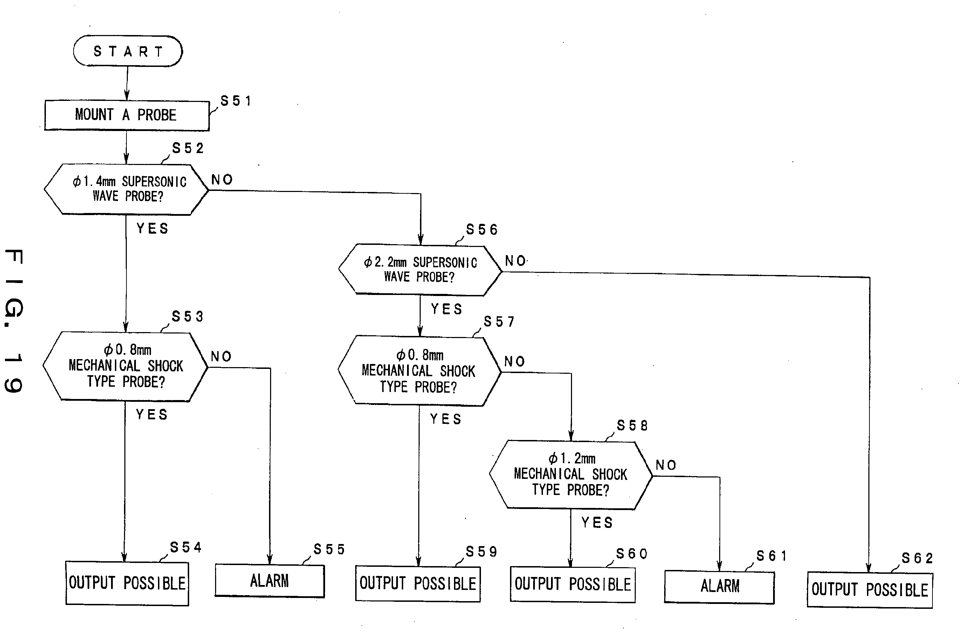

The CPU 31d stores the combined data of Table 1. The CPU 31d determines whether output is available, according to information from the RFID reader 56, as shown in Fig. 19, and the alarm unit 57 issues an alarm.

Fig. 19 is a flowchart showing that the CPU 31 determines whether the mechanical shock output of the mechanical shock type probe 24 is available against the supersonic wave probe 22 or whether to issue an alarm, according to the combined data of Table 1. As shown in Fig. 19, an operator mounts the supersonic wave probe 22 and the mechanical shock type probe 24 on the calculus breaking probe device 7D (step S51). The CPU 31d determines whether the inner diameter of the supersonic wave probe 22 is φ1.4mm, according to information from the RFID reader 56 (step S52). If the inner diameter of the supersonic wave probe 22 is φ1.4mm, the CPU 31d determines whether the outer diameter of the mechanical shock type probe 24 is φ0.8mm (step S53). If the outer diameter of the mechanical shock type probe 24 is φ0.8mm, the CPU 31d determines that mechanical shock output is available (step S54). If the outer diameter of the mechanical shock type probe 24 is not φ0.8mm, the CPU 31d controls the alarm unit 57 to issue an alarm (step S55) .

If the inner diameter of the supersonic wave probe 22 is not φ1.4mm, the CPU 31d determines whether the inner diameter of the supersonic wave probe 22 is φ2.2mm (step S56) . If the inner diameter of the supersonic wave probe 22 is φ2.2mm, the CPU 31d determines whether the outer diameter of the mechanical shock type probe 24 is φ0.8mm or φ1.2mm (step S57 or S58). If the outer diameter of the mechanical shock type probe 24 is φ0.8mm or φ1.2mm, the CPU 31d determines that mechanical shock output is available (step 59 or S60). If the outer diameter of the mechanical shock type probe 24 is neither φ0.8mm nor φ1.2mm, the CPU 31d controls the alarm unit 57 to issue an alarm (step S61).

If the inner diameter of the supersonic wave probe 22 is not φ2.2mm, the CPU 31d determines that the inner diameter of the supersonic wave probe 22 is φ3.0mm. In this case, even when the outer diameter of the mechanical shock type probe 24 is any of φ0.8mm, 1.2mm and 2.0mm, the CPU 31d determines that mechanical shock output is available (step S62).

Although in Fig. 19, it is determined whether the output of the mechanical shock type probe 24 is available against an available supersonic wave probe 22, it can also be determined whether the output of the supersonic wave probe 22 is available against an available mechanical shock type probe 24.

Thus, the calculus breaking system can determine whether supersonic wave output and mechanical shock output are available, against the combination of the supersonic wave probe 22 and the mechanical shock type probe 24 and can prevent mis-mounting and wrong output.

Against the combination of the supersonic wave probe 22 and the mechanical shock type probe 24, the output level to each probe can also be set as shown in Table 2.

**[Table 2]**

| Supersonic wave output | Mechanical shock output | Supersonic wave output | Mechanical shock output |
|---|---|---|---|
| probe *φ* 1.4mm | probe *φ* 0.8mm | Level 1 | Level 1 |
| probe *φ* 2.2mm | probe *φ* 0.8mm | Level 2 | Level 2 |
| probe *φ* 2.2mm | probe *φ* 1.2mm | Level 2 | Level 3 |
| probe *φ* 3.0mm | probe *φ* 0.8mm | Level 3 | Level 3 |
| probe *φ* 3.0mm | probe *φ* 1.2mm | Level 3 | Level 4 |
| probe *φ* 3.0mm | probe *φ* 2.0mm | Level 4 | Level 5 |

Table 2 shows the output level of the supersonic wave probe 22 and that of the mechanical shock type probe 24 in each combination of the supersonic wave probe 22 and the mechanical shock type probe 24 shown in Table 1. Table 2 particularly shows the combinations of the output levels 1-5 of the supersonic wave probe 22 and those of the mechanical shock type probe 24 in the case where three outer diameters of the mechanical shock type probe 24, φ0.8mm, 1.2mm and 2.0mm are used against three inner diameters of the supersonic wave probe 22, φ1.4mm, 2.2mm and 3.0mm. As to its level value, the larger its number, the higher its output.

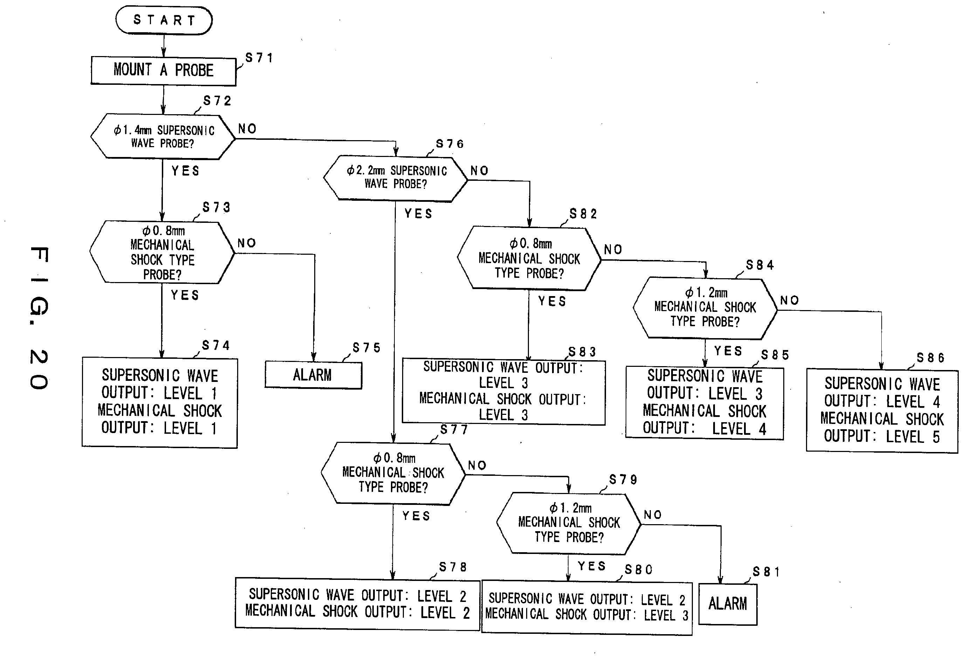

The CPU 31d stores the combined data of Table 2. The CPU 31d sets the output level of each probe, according to information from the RFID reader 56, as shown in Fig. 20.

Fig. 20 is a flowchart showing that the CPU controls the setting of each output level, according to the combined data shown in Table 2. As shown in Fig. 20, an operator mounts the supersonic wave probe 22 and the mechanical shock type probe 24 on the calculus breaking probe device 7D (step D71) . Then, the CPU 31d determines whether the inner diameter of the supersonic wave 22 is φ1.4mm, according to information from the RFID reader 56 (step S72). If the inner diameter of the supersonic wave probe 22 is φ1.4mm, the CPU 31d determines whether the outer diameter of the mechanical shock type probe 24 is φ0.8mm (step S73). If the outer diameter of the mechanical shock type probe 24 is φ0.8mm, the CPU 31d determines that mechanical shock output is available. In this case, the CPU 31d sets the each level of supersonic wave output and mechanical shock output to Level1 (step S74). If the outer diameter of the mechanical shock type probe 24 is not φ0.8mm, the CPU 31d controls the alarm unit 57 to issue an alarm (step S75) .

If the inner diameter of the supersonic wave probe 22 is not φ1.4mm, the CPU 31d determines whether the inner diameter of the supersonic wave probe 22 is φ2.2mm (step S76) . If the inner diameter of the supersonic wave probe 22 is φ2.2mm, the CPU 31d determines whether the outer diameter of the mechanical shock type probe 24 is φ0.8mm (step S77). If the outer diameter of the mechanical shock type probe 24 is φ0.8mm, the CPU 31d determines that mechanical shock output is available. In this case, the CPU 31d sets the each level of supersonic wave output and mechanical shock output to Level2 (step S78).

If the outer diameter of the mechanical shock type probe 24 is not φ0.8mm, the CPU 31d determines whether the outer diameter of the mechanical shock type probe 24 is φ1.2mm (step S79). If the outer diameter of the mechanical shock type probe 24 is φ1.2mm, the CPU 31d determines that mechanical shock output is available. In this case, the CPU 31d sets the levels of supersonic wave output and mechanical shock output to Level2 and Level3, respectively (step S80).

If the outer diameter of the mechanical shock type probe 24 is not φ1.2mm, the CPU 31d controls the alarm unit 57 to issue an alarm (step S81). If the outer diameter of the supersonic wave probe 22 is not φ2.2mm, the CPU 31d determines that the outer diameter of the supersonic wave probe 22 is φ3.0mm. Then, the CPU 31d determines whether the outer diameter of the mechanical shock type probe 24 is φ0.8mm (step S82). If the outer diameter of the mechanical shock type probe 24 is φ0.8mm, the CPU 31d sets the each level of supersonic wave output and mechanical shock output to Level3 (step S83).

If the outer diameter of the mechanical shock type probe 24 is not φ0.8mm, the CPU 31d determines whether the outer diameter of the mechanical shock type probe 24 is φ1.2mm (step S84). If the outer diameter of the mechanical shock type probe 24 is φ1.2mm, the CPU 31d determines that mechanical shock output is available. In this case, the CPU 31d sets the levels of supersonic wave output and mechanical shock output to Level3 and Level4, respectively (step S85).

If the outer diameter of the mechanical shock type probe 24 is not φ1.2mm, the CPU 31d determines that the outer diameter of the mechanical shock type probe 24 is φ2.0mm. In this case, the CPU 31d sets the levels of supersonic wave output and mechanical shock output to Level4 and Level5, respectively (step S86).

In this preferred embodiment, a supersonic wave output level and a mechanical shock output level are combined as described above. However, if taking into consideration the stability of the supersonic wave probe 22, the level of the supersonic wave probe 22 can also decrease by one to restrict the level in order to prevent the friction between the supersonic wave probe 22 and the mechanical shock type probe 24 when the difference between the inner diameter of the supersonic wave probe 22 and the outer diameter of the mechanical shock type probe 24 is a prescribed value or less, for example, 1mm or less.

Although in Fig. 20, it is determined whether the output of the mechanical shock type probe 24 is available against an available supersonic wave probe 22, it can also be determined whether the output of the supersonic wave probe 22 is available against an available mechanical shock type probe 24.

Thus, the calculus breaking system can set an output level to each probe of the combined the supersonic wave probe 22 and the mechanical shock type probe 24 and can prevent wrong output. If the inner diameter of the supersonic wave probe 22 and the outer diameter of the mechanical shock type probe 24 do not interfere with each other, the supersonic wave probe 22 and the mechanical shock type probe 24 can also be simultaneously driven.

### < The third preferred embodiment>

In the first and second preferred embodiments, supersonic wave output and mechanical shock output can be alternatively controlled. However, in the third preferred embodiment, an internal switch provided for the calculus breaking probe device 7 controls the availability of mechanical shock output. Since the other components are the same as in the first and second preferred embodiments, their descriptions are omitted here and the same reference numerals are attached to the same components.

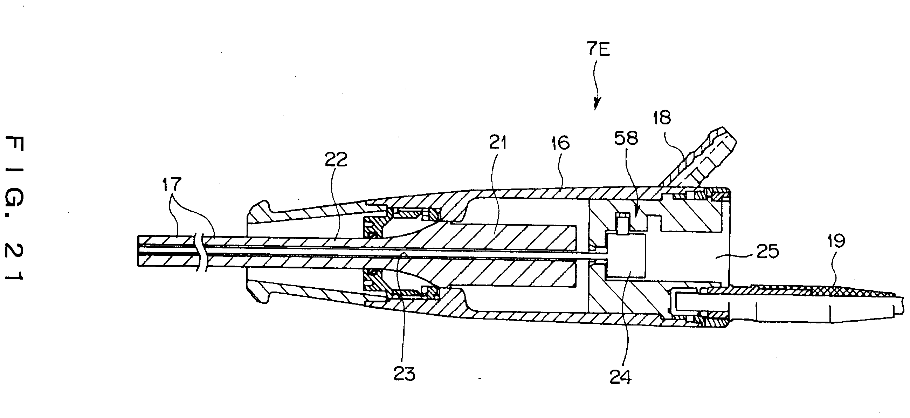

Fig. 21 is the section view of the calculus breaking probe device constituting the calculus braking system of the third preferred embodiment. As shown in Fig. 21, in a calculus breaking probe device 7E, an accommodation unit 25 accommodates the rear end of the mechanical shock type probe 24. A switching unit 58 is provided on the inner circumference side of the accommodation unit 25. The switching unit 58 controls the availability of mechanical shock output. Furthermore, the switch unit 58 is described in detail below.

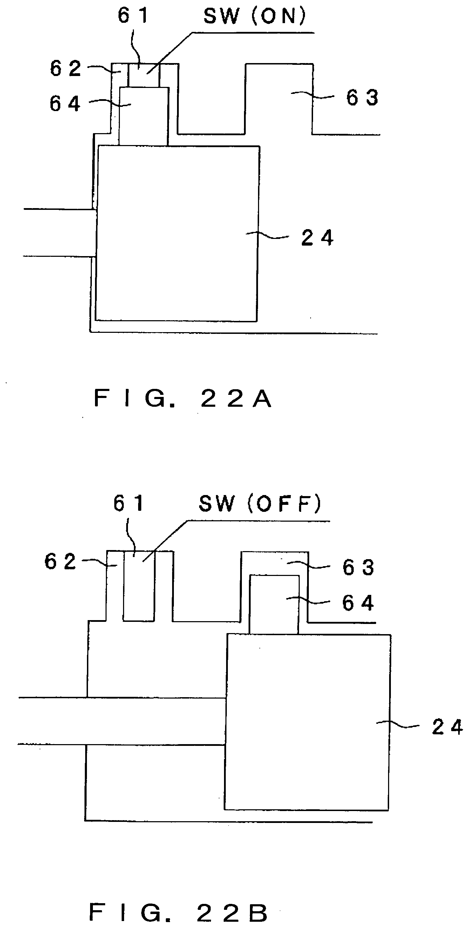

Figs. 22A and 22B are the enlarged views of the switching unit 58 shown in Fig. 21. Fig. 22A is the enlarge view of the switching unit 58 in the case where the switch is on. Fig. 22B is the enlarged view of the switching unit 28 in the case where the switch is off.

As shown in Figs. 22A and 22B, in the switching unit 58, a switch-on groove unit 62 provided with a switch (SW) 61 is formed. The switch (SW) 61 controls the availability of mechanical shock output on the inner circumference side of the accommodation unit 25. At the rear of this switch-on groove unit 62, a switch-off groove unit 63 is formed. The switch-off groove unit 63 turns the switch 61 of the switch-on groove unit 62. At the rear end of the mechanical shock type probe 24, a projection unit 64 is provided. The projection unit 64 turns the switch 61 of the switch-on groove unit 62 on.

Thus, the mechanical shock type probe 24 is retreated so as to turn the switch 61 of the switch-on groove unit 62. Then, the projection unit 64 can be placed into the switch-off groove unit 63. Thus, the calculus breaking probe device 7E can stop mechanical shock output.

Then, the mechanical shock type probe 24 is pushed forward, and the projection unit 64 is placed out of the switch-off groove unit 63 and is placed into the switch-on groove unit 62. Then, the projection unit 64 turns the switch 61 of the switch-on groove unit 62 on. Thus, the mechanical shock output of the calculus breaking probe device 7E can be made available.

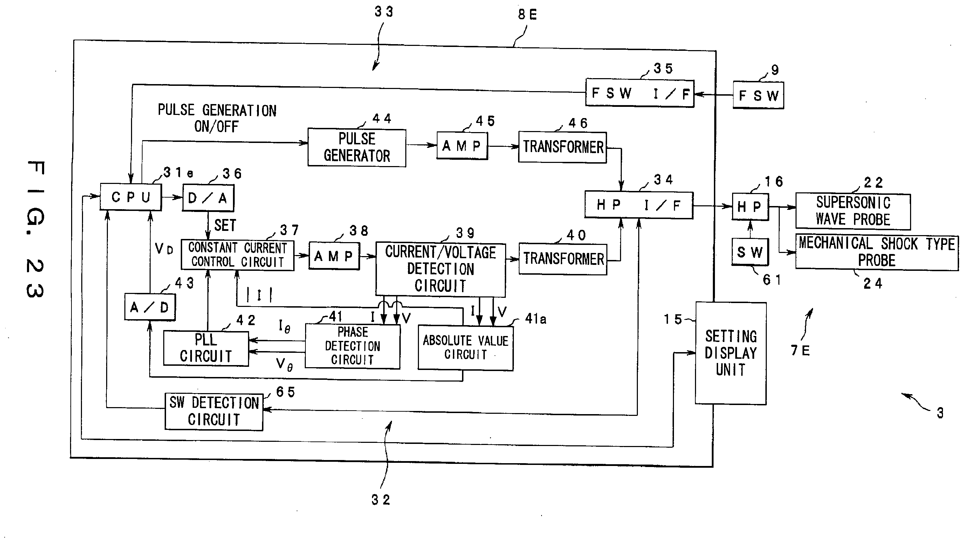

The deriving device 8E for controlling/driving the calculus breaking probe device 7E has the configuration shown in Fig. 23.

Fig. 23 is the circuit diagram of the calculus breaking device with the calculus breaking probe device shown in Fig. 21. As shown in Fig. 23, the driving device 8E comprises a switch detection circuit 65. The switch detection circuit 65 detects the on/off of the switch 61 of the calculus breaking probe device 7E.

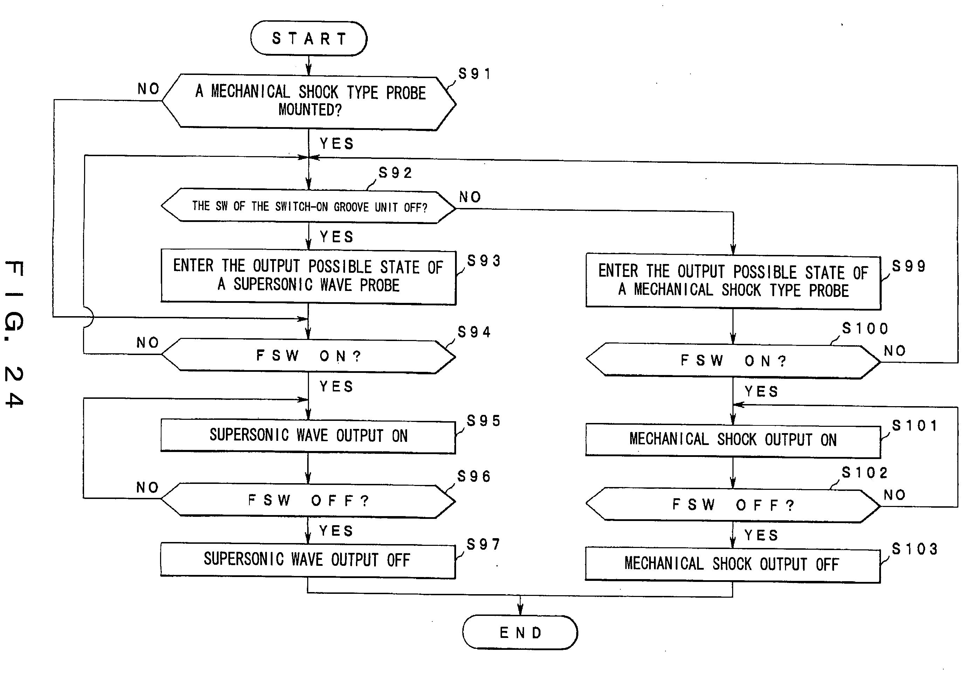

The CPU 31e switches between the output of the supersonic wave output circuit 32 and that of the mechanical shock output circuit 33 to output them to the hand piece 16, according to the flowchart shown in Fig. 24, based on the on/off signal from the switch detection circuit 65. Thus, the supersonic wave probe 22 and the mechanical shock type probe 24 are switched between and controlled. Since the other components are the same as in the first preferred embodiment, their descriptions are omitted here.

The calculus breaking system configured so is also used under an endoscope as described in the first preferred embodiment. The calculus breaking probe device 7E is led into the coelom through the treatment instrument inserting channel of the endoscope 4. Then, the calculus breaking probe device 7E breaks a calculus in the coelom under the control of the driving device 8E.

Fig. 24 is a flowchart showing the control of the CPU shown in Fig. 23. As shown in Fig. 24, firstly, the CPU 31e determines whether the mechanical shock type probe 24 is mounted (step S91). If the mechanical shock type probe 24 is mounted, the process proceeds to a subsequent step.

Then, the CPU 31e determines whether the switch 61 of the switch-on groove unit 62 is off, according to the detection result from the switch detection circuit 65 (step S92) . If the switch 61 of the switch-on groove unit 62 is off, the CPU 31e enters the output possible state of a supersonic wave in order to operate the supersonic wave probe 22 (step S93).

Then, an operator applies the tip of the insertion unit 17 of the calculus breaking probe device 7E to a calculus and operates the footswitch 9. Thus, the calculus is broken by mechanical shock output. The CPU 31e detects whether the footswitch 9 is turned on (step S94).

At this time, the supersonic wave output circuit 32 is supplying the supersonic wave probe 22 with weak current since the footswitch 9 is turned on. Although the absolute value detection circuit 41a can detect impedance, the CPU 31e can also detect the impedance as in the first preferred embodiment. If the footswitch 9 is not turned on, the CPU 31e returns to step S92.

If the impedance is equal to or less than the prescribed value T₂, the CPU 31e controls the supersonic wave output circuit 32 to turn supersonic wave output on (step S95).

When current is supplied from the driving device 8E, the supersonic vibrator 21 is driven and a supersonic wave vibration is generated. The generated supersonic wave vibration is conveyed to a probe tip and the probe tip vibrates. Then, the supersonic wave probe 22 breaks an applied calculus.

The CPU 31e keeps the supersonic wave output on and continues to drive the supersonic wave probe 22 until the footswitch 9 is turned off and the supersonic wave output is turned off (steps S96 and S97).

If the switch 61 of the switch-on groove unit 62 is turned on, the CPU 31e enters the output possible state of a mechanical shock in order to operate the mechanical shock type probe 24 (step S99).

Then, an operator applies the tip of the insertion unit 17 of the calculus breaking probe device 7E to a calculus and operates the footswitch 9. Thus, the calculus is broken by mechanical shock output.

The CPU 31e detects whether the footswitch 9 is turned on (step S100). If the footswitch 9 is turned on, the CPU 31e controls the mechanical shock output circuit 33 to turn mechanical shock output on (step S101).

When a pulse signal is supplied from the driving device 8E, the mechanical shock type probe 24 advances/retreats. Then, the mechanical shock type probe 24 applies a strong mechanical shock to the calculus to give a mechanical shock wave to it and break it. If the footswitch 9 is not turned on, the CPU 31e returns to step S92. Then, the CPU 31e keeps the mechanical shock output on and continues to drive the mechanical shock type probe 24 until the footswitch 9 is turned off and the mechanical output is turned off (steps S102 and S103).

Thus, the calculus breaking system of this preferred embodiment can obtain the same effect as in the first and second preferred embodiments. In addition to this, the availability of mechanical shock output can be controlled by the switching unit 58 provide for the calculus breaking probe device 7E. Therefore, as long as the operator does not operate the switching unit 58 intentionally, the mechanical shock type calculus breaking function of this calculus breaking system is not operated.

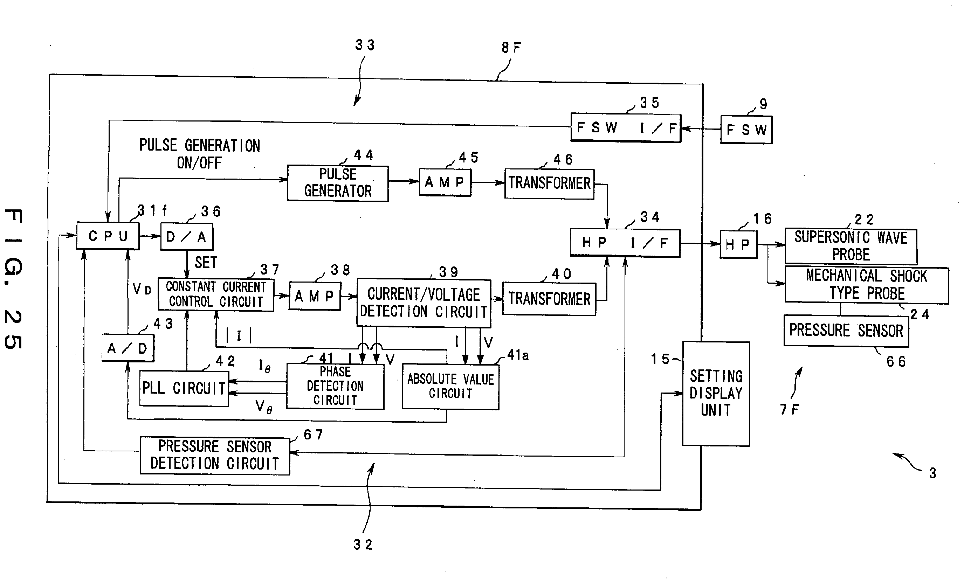

In this calculus breaking system, the mechanical shock type probe 24 can also be provided with a pressure sensor, as shown in Fig. 25, instead of providing the calculus breaking probe device 7 with the switching unit 58.

Fig. 25 is the internal circuit diagram of the variation of the calculus breaking device shown in Fig. 23. As shown in Fig. 25, in the calculus breaking probe device 7F, the mechanical shock type probe 24 is provided with a pressure sensor 66. The pressure sensor 66 senses a pressure applied to a calculus when applying the mechanical shock type probe to it.

The driving device 8F for controlling/driving the calculus breaking probe device 7F comprises a pressure sensor detection circuit 67. The pressure sensor detection circuit 67 receives a signal from the pressure sensor 66 provided for the mechanical shock type probe 24 and detects a pressure.

Then, a CPU 31f compares the pressure with a prescribed value T₃, which is a preset threshold value, according to the detection result from the pressure sensor detection circuit 67 and determines whether the pressure exceeds this prescribed value T₃. Then, the CPU 31f controls the mechanical shock output circuit 33, according to the determination result.

The calculus breaking system configured so is also used under an endoscope as described in the first preferred embodiment. The calculus breaking probe device 7F is led into the coelom through the treatment instrument inserting channel of the endoscope 4. Then, the calculus breaking probe device 7F breaks a calculus in the coelom under the control of the driving device 8F.

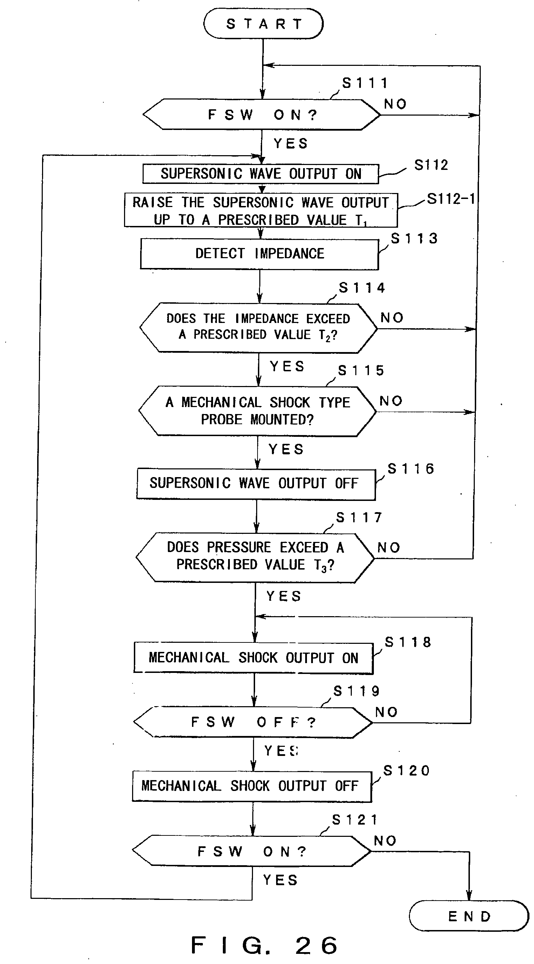

Then, an operator applies the tip of the insertion unit 17 of the calculus breaking probe device 7F to a calculus and operates the footswitch 9. Thus, the calculus is broken according to the flowchart shown in Fig. 26.

Fig. 26 is a flowchart showing the control of the CPU shown in Fig. 25. As shown in Fig. 26, firstly, the CPU 31f detects whether the footswitch 9 is turned on (step Sill). If the footswitch 9 is on, the CPU 31f controls the supersonic wave output 32 to turn supersonic wave output on (step S112).

When current is supplied from the driving device 8F, the supersonic wave vibrator 21 is driven and a supersonic wave vibration is generated. The generated supersonic wave vibration is conveyed to a probe tip and the probe tip vibrates. Thus, the supersonic wave probe 22 breaks an applied calculus. If the footswitch 9 is not turned on, the CPU 31f returns to step S111.

Then, the supersonic wave output (output level) gradually rises up to a prescribed value T₁, which is a preset threshold value, under the control of the CPU 31f (step S112-1). After the supersonic wave output rises up to the prescribed value T₁, the absolute value detection circuit 41a detects (calculates) an impedance (step S113). The CPU 31f determines whether the impedance detected by the absolute value detection circuit 41a exceeds a prescribed value T₂ (step S114) . The CPU 31f determines whether the mechanical shock type probe 24 is mounted, according to the operator's setting of the setting display unit 15 (step S115) . If the impedance exceeds the prescribed value T₂ and also the mechanical shock type probe 24 is mounted, the CPU 31f controls the supersonic wave output circuit 32to turn supersonic wave output off (step S116). Then, the calculus breaking probe device 7F stop the supersonic wave vibration of the supersonic wave probe 22 (turn it off). If the impedance is equal to or less than the prescribed value T₂ and also the mechanical shock type probe 24 is not mounted, the CPU 31f returns to step S111.

The calculus breaking probe device 7F detects a pressure applied to a calculus by the pressure sensor 66 in a state where the supersonic wave vibration stops. A signal from the pressure sensor 66 is outputted to the pressure sensor detection circuit 67 of the driving device 8F. The pressure sensor detection circuit 67 outputs the detection result to the CPU 31f.

Then, the CPU 31f determines whether the pressure exceeds the prescribed value T₃, according to the detection result from the pressure sensor detection circuit 67 (step S117). If the pressure exceeds the prescribed value T₃, the CPU 31f controls the mechanical shock output circuit33 to turn mechanical shock output on (step S118) .

When a pulse signal is supplied from the driving device 8F, the mechanical shock type probe 24 advances/retreats. Then, the mechanical shock type probe 24 applies a strong mechanical shock to a calculus to give a mechanical shock wave to it and to break it.

Then, the CPU 31f continues the mechanical shock output until the footswitch 9 is turned off (step S119) and the mechanical shock output is turned off (step S120) . When the footswitch 9 is turned off, the CPU 31f turns the mechanical shock output off. When the operator turns the footswitch on again, the CPU 31f returns to step S112 and repeats S112 through S121.

Thus the variation of the calculus breaking system can obtain the same effect as in the third preferred embodiment. In addition to this, since it can control the availability of mechanical shock output by the pressure sensor 66 provided for the mechanical shock type probe 24, this calculus breaking system can automatically operate the mechanical shock type calculus breaking function.

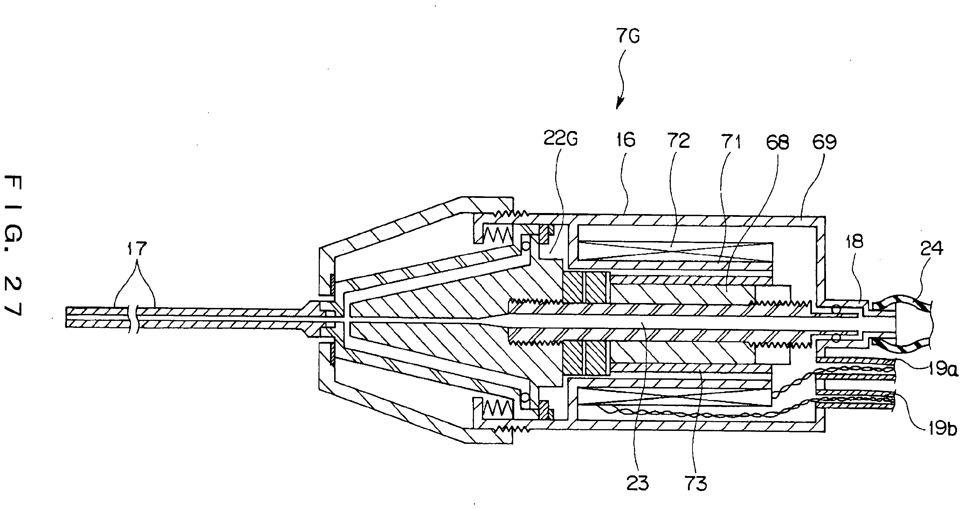

If the mechanical shock output of the calculus breaking probe device 7 is not large and a shock to a calculus is not strong, as shown in Fig. 27, the supersonic wave probe can also be used as the mechanical shock type probe.

Fig. 27 shows the configuration of the calculus breaking probe device in which a supersonic wave probe is also used as a mechanical shock type probe. As shown in Fig. 27, in a calculus breaking probe device 7G, a supersonic wave probe 22G is also used as a mechanical shock type probe.

The calculus breaking probe device 7G has a Langevin type vibrator 68 inside the hand piece 16 as a supersonic wave vibrator. In the insertion unit 17, the supersonic wave probe 22G extended from this Langevin type vibrator 68 projects from the opening.

In the calculus breaking probe device 7G, a support unit 71 is formed and incorporated into an armoring material 69 away off the outside of the Langevin type vibrator 68 in side the hand piece 16. In the circumference of this support unit 71, a coil 72 for generating a magnetic field is provided. A reference numeral 19a represents a cable mouthpiece for supplying the coil 72 with current. A reference numeral 19b represents a cable mouthpiece for supplying the Langevin type vibrator 68 with current.

In the supersonic wave probe 22G, a metal frame 73 is joined around the outer circumference of the Langevin type vibrator 68 so as to be affected by the magnetic field of the coil 72. When outputting a supersonic wave, if in the calculus breaking probe device 7G, current is supplied from the driving device 8, the Langevin type vibrator 68 is driven and a supersonic wave vibration is generated. The generated supersonic wave vibration is conveyed to a probe tip and the probe tip is vibrated by a supersonic wave. Thus, the calculus breaking probe device 7G breaks an applied calculus.

When outputting a mechanical shock, if in the calculus breaking probe device 7G, current is supplied from the driving device 8, the coil 72 generates a magnetic field. The generated magnetic field acts on the metal frame 73 of the Langevin type vibrator 68 to advance/retreat the entire supersonic wave probe 22G. Thus, the calculus breaking probe device 7G gives a mechanical shock to an applied calculus.

Therefore, if the shock to the calculus is not strong, the supersonic wave probe 22G can be also used as the mechanical shock type probe 24.

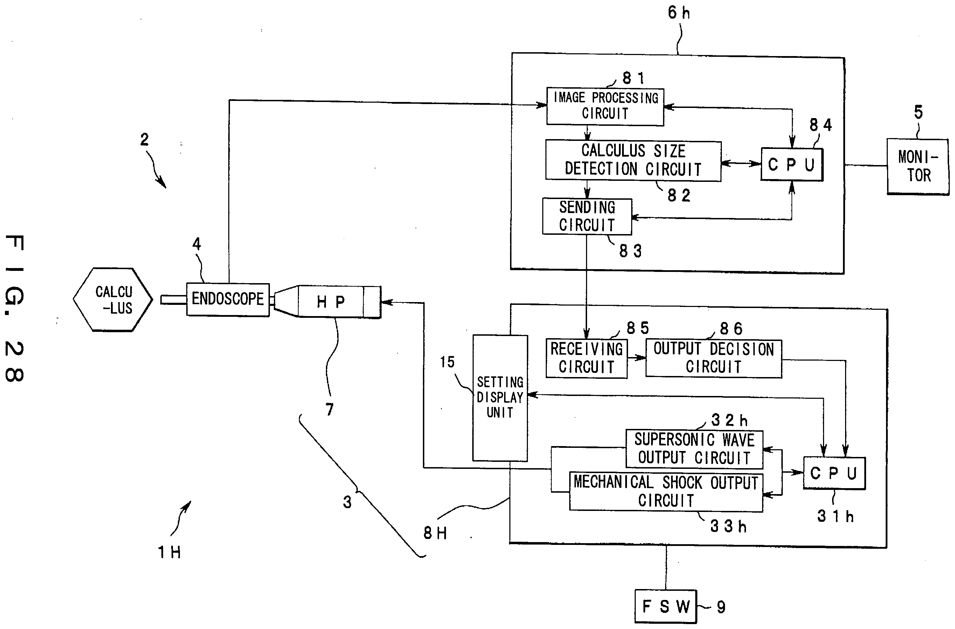

The calculus breaking system can also detect the size of a calculus from an endoscopic image as shown in Fig. 28, and can also switch between and control the supersonic wave probe 22 and the mechanical shock type probe 24.

Fig. 28 shows the circuit diagram of the calculus breaking system for switching between and controlling a supersonic wave probe and a mechanical shock type probe, according to a calculus size detected from an endoscopic image. As shown in Fig. 28, a calculus breaking system 1H comprises a CCU 6h. The CCU 6h comprises a video processing circuit 81, a calculus size detecting circuit 82, a transmitting circuit 83 and a CPU 84.

The calculus size detecting circuit 82 detects a calculus size from a video signal obtained by processing an image using the video processing circuit 81. The transmitting circuit 83 transmits the detection result of the calculus size detecting circuit 82.

A driving device 8H comprises a supersonic wave output circuit 32h, a mechanical shock output circuit 33h, a receiving circuit 85 and an output determination circuit 86. The receiving circuit 85 receives the detection result of a calculus size from the CCU 6h. The output determination circuit 86 determines which to output, a supersonic wave from the supersonic wave output circuit 32h or a mechanical shock from a mechanical shock from the mechanical shock output circuit 33h, according to the detection result from the receiving circuit 85. The output determination circuit 86 outputs the determination result to a CPU 31h.

Then, the driving device 8H switches between output from the supersonic wave output circuit 32h and output from the mechanical shock output circuit 33h, under the control of the CPU 31h, according to a flowchart, which is described later, and output it to the hand piece 16. Thus, the driving device 8H switches between/controls the supersonic wave probe 22 and the mechanical shock type probe 24.

The calculus breaking system 1H configured so is also used under an endoscope as described in the first preferred embodiment. The calculus breaking probe device 7F is led into the coelom through the treatment instrument inserting channel of the endoscope 4. Thus, the calculus breaking probe device 7F breaks a calculus in the coelom under the control of the driving device 8F.

In this case, the endoscope 4 outputs a camera signal obtained by shooting an endoscopic image by a camera unit, which is not shown in Fig. 28, to the CCU 6h. In the CCU 6h, the video processing circuit 81 generates a video signal from the camera signal. Then, the CCU 6h displays the Endoscopic image on a monitor 5. Simultaneously, the calculus size detecting circuit 82 detects a calculus size from the video signal from the video processing circuit 81. The CCU 6h transmits this detection result to the driving device 8H via the transmitting circuit 83.

The driving device 8H receives the calculus size detection result by the receiving circuit 85. According to this received detection result, the output determination circuit 86 determines which to output, a supersonic wave from the supersonic wave output circuit 32h or a mechanical shock from the mechanical shock output circuit 33h. Then, the output determination circuit 86 outputs this determination result to the CPU 31h.

Then, the CPU 31h of the driving device 8H switches between/controls a transition to the output possible state of the supersonic wave probe 22 and a transition to the output possible state of the mechanical shock type probe 24.

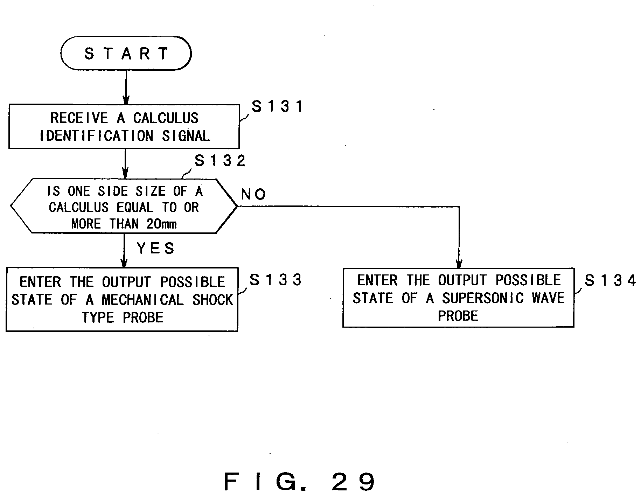

Fig. 29 is a flowchart showing the control of the CPU shown in Fig. 28, for switching between and controlling entering the respective output possible states of a supersonic wave probe and a mechanical shock type probe, according to a calculus size.

As shown in Fig. 29, the CPU 31h receives a calculus identification signal from an output determination circuit 86 (step S131). The CPU 31h determines whether the length of a calculus side is equal to or more than, for example, 20mm (step S132) . If the length of a calculus side exceeds 20mm, the CPU 31h enters the output possible state of the mechanical shock type probe 24 (step S132) . If the length of a calculus side is less than 20mm, the CPU 31h enters the output possible state of the supersonic wave probe 22 (step S133) .

Then, the operator applies the tip of the insertion unit 17 of the calculus breaking probe device 7H and operates the footswitch 9. Thus, a calculus is broken by either the supersonic wave probe 22 or the mechanical shock type probe 24.

Thus, since the variation of the calculus breaking system 1H can enter the output possible state of either the supersonic wave probe 22 or the mechanical shock type probe 24, according to the detected calculus size, a calculus can be more easily broken.

Preferred embodiments obtained by partially combining the above-described preferred embodiment also belong to the present invention.

As described above, the calculus breaking device of the present invention can simultaneously control both a supersonic wave calculus braking function and a mechanical shock type calculus breaking function rapidly and safely.

## Claims

1. A calculus breaking device, comprising:
an ultrasonic wave probe (17, 22) for generating an ultrasonic wave vibration for breaking a calculus;
a mechanical shock type probe (24) for generating a mechanical shock wave;
a control unit (31) for switching between the ultrasonic wave probe and the mechanical shock type probe to control and drive them; and
a detection unit (39, 82) for detecting a state including hardness or a size of the calculus,
wherein
the control unit is able to switch between the ultrasonic wave probe and the mechanical shock type probe to control and drive them, according to the detection result of the detection unit.

2. The calculus breaking device according to claim 1, further comprising:
an ultrasonic wave output unit (32) for supplying a first output to drive the ultrasonic wave probe; and
a mechanical shock type output unit (33) for supplying a second output different from the first output to drive the mechanical shock type probe, wherein
the control unit (31) is able to switch between the ultrasonic wave output unit and the mechanical shock type output unit to control and drive the ultrasonic wave probe and the mechanical shock type probe.

3. The calculus breaking device according to claim 1, further comprising a time management unit (52) for managing output time to the ultrasonic wave probe,
wherein
the control unit (31) is able to switch between the ultrasonic wave probe and the mechanical shock type probe to control and drive them, according to the management of the time management unit.

4. The calculus breaking device according to claim 1, further comprising
a switching unit (47) for controlling the output possible state to the mechanical shock type probe,
wherein
the control unit (31) is able to switch between the ultrasonic wave probe and the mechanical shock type probe to control and drive them, according to the on/off of the switching unit.

5. The calculus breaking device according to claim 1, wherein
the mechanical shock type probe (24) is inserted in and disposed at the pipe of the ultrasonic wave probe, and
when it is the mechanical shock type probe breaking the calculus, the tip of the mechanical shock type probe projects from the tip of the ultrasonic wave probe.

6. The calculus breaking device according to claim 1, wherein
the control unit (31) is able to identify the respective types of the ultrasonic wave probe and the mechanical shock type probe and to determine the respective output possible states to the ultrasonic wave probe and the mechanical shock type probe.

7. The calculus breaking device according to claim 1, wherein
the control unit (31) is able to identify the respective types of the ultrasonic wave probe and the mechanical shock type probe and to set the respective output levels to the ultrasonic wave probe and the mechanical shock type probe.

8. The calculus breaking device according to claim 1,
further comprising:
a pressure detection unit (67) for detecting a pressure when applying the mechanical shock type probe (24) to the calculus; wherein
the control unit (31) is able to control and drive the mechanical shock type probe, when detecting a pressure by the pressure detection unit when applying the mechanical shock type probe to the calculus.

9. The calculus breaking device according to claim 1, wherein
the detection unit (39, 82) is able to detect output to the ultrasonic wave shock type probe as information indicating the state of the calculus, and
the control unit (31) is able to switch between the ultrasonic wave probe and the mechanical shock type probe to control and drive them, according to the output detected by the detection unit.

10. The calculus breaking device according to claim 1, wherein
the detection unit (39, 82) is able to detect output time to the ultrasonic wave probe as information indicating the state of the calculus and
the control unit (31) is able to switch between the ultrasonic wave probe and the mechanical shock type probe to control and drive them, according to the output time detected by the detection unit.

11. The calculus breaking device according to claim 1, wherein
the detection unit (39, 82) is able to detect the size of a calculus as information indicating the state of the calculus and
the control unit (31) is able to switch switches between the ultrasonic wave probe and the mechanical shock type probe to control and drive them, according to the calculus size detected by the detection unit.

12. The calculus breaking device according to claim 5, wherein
the position of the tip of the mechanical shock type probe is matched with the position of the tip of the ultrasonic wave probe.

13. The calculus breaking device according to claim 5, wherein
the position of the tip of the mechanical shock type probe is behind the position of the tip of the ultrasonic wave probe by almost a half of the stroke.

14. The calculus breaking device according to claim 5, further comprising
a driving unit (53) for advancing and retreating the tip of the mechanical shock type probe against the tip of the ultrasonic wave probe freely both in a projected and retreated state,
wherein
the control unit (31) is able to advance and retreat the mechanical shock type probe by controlling the driving unit.

15. The calculus breaking device according to claim 1, further comprising:
a switching unit (34) for switching between the ultrasonic wave probe and the mechanical shock type probe; and
a control unit (31) switching between the ultrasonic wave probe and the mechanical shock type probes by the switching unit to control and drive them.

## Patentansprüche

1. Konkrementzertrümmerungsvorrichtung, umfassend:
eine Ultraschallwellensonde (17, 22) zum Erzeugen einer Ultraschallwellenschwingung zur Zertrümmerung eines Konkrements;
eine mechanische Stoßwellensonde (24) zur Erzeugung einer mechanischen Stoßwelle;
eine Steuereinheit (31) zum Umschalten zwischen der Ultraschallwellensonde und der mechanischen Stoßwellensonde zu deren Steuerung und Antrieb; und
eine Detektionseinheit (39, 82) zur Detektion eines Zustands, einschließlich Härte oder Größe des Konkrements, wobei die Steuereinheit fähig ist, zwischen der Ultraschallwellensonde und der mechanischen Stoßwellensonde umzuschalten, um diese entsprechend dem Detektionsergebnis der Detektionseinheit zu steuern und anzutreiben.

2. Konkrementzertrümmerungsvorrichtung nach Anspruch 1, weiterhin umfassend:
eine Ultraschallwellen-Ausgabeeinheit (32) zur Bereitstellung einer ersten Ausgabe zum Antrieb der Ultraschallwellensonde; und
eine mechanische Stoßwellen-Ausgabeeinheit (33) zur Bereitstellung einer zweiten, von der ersten Ausgabe verschiedenen Ausgabe zum Antrieb der mechanischen Stoßwellensonde, wobei
die Steuereinheit (31) fähig ist, zwischen der Ultraschallwellen-Ausgabeeinheit und der mechanischen Stoßwellen-Ausgabeeinheit umzuschalten, um die Ultraschallwellensonde und die mechanische Stoßwellensonde zu steuern und anzutreiben.

3. Konkrementzertrümmerungsvorrichtung nach Anspruch 1, weiterhin umfassend:
eine Zeitverwaltungseinheit (52) zur Verwaltung der Ausgabezeit für die Ultraschallwellensonde, wobei
die Steuereinheit (31) fähig ist, zwischen der Ultraschallwellensonde und der mechanischen Stoßwellensonde umzuschalten, um diese gemäß der Verwaltung der Zeitverwaltungseinheit zu steuern und anzutreiben.

4. Konkrementzertrümmerungsvorrichtung nach Anspruch 1, weiterhin umfassend:
eine Umschalteinheit (47) zur Steuerung des Ausgabe-möglich-Zustands für die mechanische Stoßwellensonde, wobei
die Steuereinheit (31) fähig ist, zwischen der Ultraschallwellensonde und der mechanischen Stoßwellensonde umzuschalten, um diese gemäß dem Ein/Aus der Umschalteinheit zu steuern und anzutreiben.

5. Konkrementzertrümmerungsvorrichtung nach Anspruch 1, wobei
die mechanische Stoßwellensonde (24) im Rohr der Ultraschallwellensonde eingeführt und dort angeordnet ist und
wenn das Konkrement durch die mechanische Stoßwellensonde zertrümmert wird, die Spitze der mechanischen Stoßwellensonde von der Spitze der Ultraschallwellensonde übersteht.

6. Konkrementzertrümmerungsvorrichtung nach Anspruch 1, wobei
die Steuereinheit (31) fähig ist, die entsprechenden Typen der Ultraschallwellensonde und der mechanischen Stoßwellensonde zu identifizieren und die entsprechenden Ausgabe-möglich-Zustände für die Ultraschallwellensonde und die mechanische Stoßwellensonde zu bestimmen.

7. Konkrementzertrümmerungsvorrichtung nach Anspruch 1, wobei
die Steuereinheit (31) fähig ist, die entsprechenden Typen der Ultraschallwellensonde und der mechanischen Stoßwellensonde zu identifizieren und die entsprechenden Ausgabestufen für die Ultraschallwellensonde und die mechanische Stoßwellensonde einzustellen.

8. Konkrementzertrümmerungsvorrichtung nach Anspruch 1,
weiterhin umfassend:
eine Druckdetektionseinheit (67) zur Detektion eines Drucks bei Anwendung der mechanischen Stoßwellensonde (24) auf das Konkrement; wobei
die Steuereinheit (31) fähig ist, die mechanische Stoßwellensonde zu steuern und anzutreiben, wenn ein Druck durch die Druckdetektionseinheit bei der Anwendung der mechanischen Stoßwellensonde auf das Konkrement detektiert wird.

9. Konkrementzertrümmerungsvorrichtung nach Anspruch 1, wobei
die Detektionseinheit (39, 82) fähig ist, eine Ausgabe für die Ultraschallwellen-Stoßwellensonde als Information zu detektieren, die den Zustand des Konkrements anzeigt, und
die Steuereinheit (31) fähig ist, zwischen der Ultraschallwellensonde und der mechanischen Stoßwellensonde umzuschalten, um diese gemäß der von der Detektionseinheit detektierten Ausgabe zu steuern und anzutreiben.

10. Konkrementzertrümmerungsvorrichtung nach Anspruch 1, wobei
die Detektionseinheit (39, 82) fähig ist, eine Ausgabezeit für die Ultraschallwellensonde als Information zu detektieren, die den Zustand des Konkrements anzeigt, und
die Steuereinheit (31) fähig ist, zwischen der Ultraschallwellensonde und der mechanischen Stoßwellensonde umzuschalten, um diese gemäß der von der Detektionseinheit detektierten Ausgabezeit zu steuern und anzutreiben.

11. Konkrementzertrümmerungsvorrichtung nach Anspruch 1, wobei
die Detektionseinheit (39, 82) fähig ist, die Größe eines Konkrements als Information zu detektieren, die den Zustand des Konkrements anzeigt, und
die Steuereinheit (31) fähig ist, zwischen der Ultraschallwellensonde und der mechanischen Stoßwellensonde umzuschalten, um diese gemäß der von der Detektionseinheit detektierten Konkrementgröße zu steuern und anzutreiben.

12. Konkrementzertrümmerungsvorrichtung nach Anspruch 5, wobei
die Position der Spitze der mechanischen Stoßwellensonde mit der Position der Spitze der Ultraschallwellensonde abgestimmt wird.

13. Konkrementzertrümmerungsvorrichtung nach Anspruch 5, wobei
die Position der Spitze der mechanischen Stoßwellensonde um fast eine Hälfte des Hubs hinter der Position der Spitze der Ultraschallwellensonde liegt.

14. Konkrementzertrümmerungsvorrichtung nach Anspruch 5, weiterhin umfassend:
eine Antriebseinheit (53) zum freien Vorschub und Rückzug der Spitze der mechanischen Stoßwellensonde gegen die Spitze der Ultraschallwellensonde sowohl in einen ausgefahrenen als auch einen eingefahrenen Zustand, wobei
die Steuereinheit (31) fähig ist, die mechanische Stoßwellensonde durch Steuerung der Antriebseinheit vorzuschieben und einzuziehen.

15. Konkrementzertrümmerungsvorrichtung nach Anspruch 1, weiterhin umfassend:
eine Umschalteinheit (34) zum Umschalten zwischen der Ultraschallwellensonde und der mechanischen Stoßwellensonde; und
eine Steuereinheit (31) zum Umschalten zwischen der Ultraschallwellensonde und den mechanischen Stoßwellensonden durch die Umschalteinheit zu deren Steuerung und Antrieb.

## Revendications

1. Dispositif de destruction de calcul, comprenant :
une sonde à onde ultrasonore (17, 22) pour générer une vibration à onde ultrasonore pour détruire un calcul ;
une sonde de type à choc mécanique (24) pour générer une onde de choc mécanique ;
une unité de commande (31) pour commuter entre la sonde à onde ultrasonore et la sonde de type à choc mécanique pour les commander et les entraîner ; et
une unité de détection (39, 82) pour détecter un état comprenant la dureté ou une taille du calcul,
l'unité de commande étant apte à commuter entre la sonde à onde ultrasonore et la sonde de type à choc mécanique pour les commander et les entraîner, selon le résultat de détection de l'unité de détection.

2. Dispositif de destruction de calcul selon la revendication 1, comprenant en outre :
une unité de délivrance d'onde ultrasonore (32) pour fournir une première sortie pour entraîner la sonde à onde ultrasonore ; et
une unité de délivrance de type à choc mécanique (33) pour fournir une seconde sortie différente de la première sortie pour entraîner la sonde de type à choc mécanique,
l'unité de commande (31) étant apte à commuter entre l'unité de délivrance d'onde ultrasonore et l'unité de délivrance de type à choc mécanique pour commander et entraîner la sonde à onde ultrasonore et la sonde de type à choc mécanique.

3. Dispositif de destruction de calcul selon la revendication 1, comprenant en outre une unité de gestion de temps (52) pour gérer le temps de sortie sur la sonde à onde ultrasonore,
l'unité de commande (31) étant apte à commuter entre la sonde à onde ultrasonore et la sonde de type à choc mécanique pour les commander et les entraîner, selon la gestion de l'unité de gestion de temps.

4. Dispositif de destruction de calcul selon la revendication 1, comprenant en outre une unité de commutation (47) pour commander l'état possible de sortie sur la sonde de type à choc mécanique,
l'unité de commande (31) étant apte à commuter entre la sonde à onde ultrasonore et la sonde de type à choc mécanique pour les commander et les entraîner, selon l'état sous tension/hors tension de l'unité de commutation.

5. Dispositif de destruction de calcul selon la revendication 1, dans lequel
la sonde de type à choc mécanique (24) est introduite dans et disposée au niveau du tuyau de la sonde à onde ultrasonore, et
lorsque la destruction du calcul est réalisée par la sonde de type à choc mécanique, l'extrémité de la sonde de type à choc mécanique fait saillie à partir de l'extrémité de la sonde à onde ultrasonore.

6. Dispositif de destruction de calcul selon la revendication 1, dans lequel
l'unité de commande (31) est apte à identifier les types respectifs de la sonde à onde ultrasonore et de la sonde de type à choc mécanique et à déterminer les états possibles de sortie respectifs sur la sonde à onde ultrasonore et la sonde de type à choc mécanique.

7. Dispositif de destruction de calcul selon la revendication 1, dans lequel
l'unité de commande (31) est apte à identifier les types respectifs de la sonde à onde ultrasonore et de la sonde de type à choc mécanique et à régler les niveaux de sortie respectifs sur la sonde à onde ultrasonore et la sonde de type à choc mécanique.

8. Dispositif de destruction de calcul selon la revendication 1, comprenant en outre :
une unité de détection de pression (67) pour détecter une pression lors de l'application de la sonde de type à choc mécanique (24) sur le calcul ;
l'unité de commande (31) étant apte à commander et à entraîner la sonde de type à choc mécanique, lors de la détection d'une pression par l'unité de détection de pression lors de l'application de la sonde de type à choc mécanique sur le calcul.

9. Dispositif de destruction de calcul selon la revendication 1, dans lequel
l'unité de détection (39, 82) est apte à détecter une sortie sur la sonde de type à choc d'onde ultrasonore en tant qu'informations indiquant l'état du calcul, et
l'unité de commande (31) est apte à commuter entre la sonde à onde ultrasonore et la sonde de type à choc mécanique pour les commander et les entraîner, selon la sortie détectée par l'unité de détection.

10. Dispositif de destruction de calcul selon la revendication 1, dans lequel
l'unité de détection (39, 82) est apte à détecter le temps de sortie sur la sonde à onde ultrasonore en tant qu'informations indiquant l'état du calcul, et
l'unité de commande (31) est apte à commuter entre la sonde à onde ultrasonore et la sonde de type à choc mécanique pour les commander et les entraîner, selon le temps de sortie détecté par l'unité de détection.

11. Dispositif de destruction de calcul selon la revendication 1, dans lequel
l'unité de détection (39, 82) est apte à détecter la taille d'un calcul en tant qu'informations indiquant l'état du calcul, et
l'unité de commande (31) est apte à commuter entre la sonde à onde ultrasonore et la sonde de type à choc mécanique pour les commander et les entraîner, selon la taille de calcul détectée par l'unité de détection.

12. Dispositif de destruction de calcul selon la revendication 5, dans lequel
la position de l'extrémité de la sonde de type à choc mécanique est mise en correspondance avec la position de l'extrémité de la sonde à onde ultrasonore.

13. Dispositif de destruction de calcul selon la revendication 5, dans lequel
la position de l'extrémité de la sonde de type à choc mécanique est derrière la position de l'extrémité de la sonde à onde ultrasonore de presque la moitié de la course.

14. Dispositif de destruction de calcul selon la revendication 5, comprenant en outre
une unité d'entraînement (53) pour faire avancer et reculer l'extrémité de la sonde de type à choc mécanique par rapport à l'extrémité de la sonde à onde ultrasonore librement à la fois dans un état projeté et un état reculé,
l'unité de commande (31) étant apte à faire avancer et reculer la sonde de type à choc mécanique par commande de l'unité d'entraînement.

15. Dispositif de destruction de calcul selon la revendication 1, comprenant en outre :
une unité de commutation (34) pour commuter entre la sonde à onde ultrasonore et la sonde de type à choc mécanique ; et
une unité de commande (31) pour réaliser une commutation entre la sonde à onde ultrasonore et la sonde de type à choc mécanique par l'unité de commutation pour les commander et les entraîner.
